# EUROPEAN PATENT SPECIFICATION

(11) **EP 4 248 896 B1**
(45) Date of publication and mention of the grant of the patent: **02.12.2026**
(21) Application number: 23164192.9
(22) Date of filing: 24.03.2023
(51) Int. Cl.: A61B 18/14

(54) **EXPANDABLE BASKET ASSEMBLIES WITH LINEAR SPINE PATTERNS FOR IMPROVED TISSUE CONTACT AND METHODS FOR MAKING THEREOF**
EXPANDIERBARE KORBANORDNUNGEN MIT LINEAREN WIRBELSÄULENMUSTERN FÜR VERBESSERTEN GEWEBEKONTAKT UND VERFAHREN ZUR HERSTELLUNG DAVON
ENSEMBLES PANIER EXTENSIBLES À MOTIFS DE COLONNE VERTÉBRALE LINÉAIRES POUR UN CONTACT TISSULAIRE AMÉLIORÉ ET LEURS PROCÉDÉS DE FABRICATION

(30) Priority: 25.03.2022 US 202263323849 P; 24.02.2023 US 202318174409
(43) Date of publication of application: 27.09.2023
(60) Divisional application: 26169614.0 / 4 778 482
(73) Proprietor: BIOSENSE WEBSTER (ISRAEL) LTD., 2066717 Yokneam (IL)
(72) Inventor: OKARSKI, Kevin Mark, Irvine, 92618 (US); DATTA, Keshava, Irvine, 92618 (US); BAH, Abubakarr, Irvine, 92618 (US); NGUYEN, Thanh, Irvine, 92618 (US)
(74) Representative: Carpmaels & Ransford LLP

(56) References cited:
- WO-A1-2020/251857
- US-A1- 2002 198 522
- US-A1- 2014 336 494
- US-A1- 2015 223 757
- US-A1- 2015 351 652
- US-A1- 2017 319 140

## Description

### FIELD

The present invention provides expandable basket assemblies for medical probes, in accordance with the appended claims. The disclosure relates generally to medical devices, and in particular catheters with expandable basket assemblies and electrodes, and further relates to, but not exclusively, catheters suitable for use to induce irreversible electroporation (IRE) of cardiac tissues.

### BACKGROUND

Cardiac arrhythmias, such as atrial fibrillation (AF), occur when regions of cardiac tissue abnormally conduct electric signals to adjacent tissue. This disrupts the normal cardiac cycle and causes asynchronous rhythm. Certain procedures exist for treating arrhythmia, including surgically disrupting the origin of the signals causing the arrhythmia and disrupting the conducting pathway for such signals. By selectively ablating cardiac tissue by application of energy via a catheter, it is sometimes possible to cease or modify the propagation of unwanted electrical signals from one portion of the heart to another.

Many current ablation approaches in the art tend to utilize radiofrequency (RF) electrical energy to heat tissue. RF ablation can have certain rare drawbacks due to operator's skill, such as heightened risk of thermal cell injury which can lead to tissue charring, burning, steam pop, phrenic nerve palsy, pulmonary vein stenosis, and esophageal fistula. Cryoablation is an alternative approach to RF ablation that generally reduces thermal risks associated with RF ablation but may present tissue damage due to the very low temperature nature of such devices. Maneuvering cryoablation devices and selectively applying cryoablation, however, is generally more challenging compared to RF ablation; therefore cryoablation is not viable in certain anatomical geometries which may be reached by electrical ablation devices.

Some ablation approaches use irreversible electroporation (IRE) to ablate cardiac tissue using nonthermal ablation methods. IRE delivers short pulses of high voltage to tissues and generates an unrecoverable permeabilization of cell membranes. Delivery of IRE energy to tissues using multi-electrode catheters was previously proposed in the patent literature. Examples of systems and devices configured for IRE ablation are disclosed in U.S. Patent Pub. No. 2021/0169550A1, 2021/0169567A1, 2021/0169568A1, 2021/0161592A1, 2021/0196372A1, 2021/0177503A1, 2021/0186604A1, 2021/0162210, and 2021/0077180.

Regions of cardiac tissue can be mapped by a catheter to identify the abnormal electrical signals. The same or different catheter can be used to perform ablation. Some example catheters include a number of spines with electrodes positioned thereon. The electrodes are generally attached to the spines and secured in place by soldering, welding, or using an adhesive. Furthermore, multiple linear spines are generally assembled together by attaching both ends of the linear spines to a tubular shaft (e.g., a pusher tube) to form a spherical basket. U.S. Patent Pub. No. 2014/0336494 A1 describes electrode support structure assemblies. U.S. Patent Pub. No. 2015/0223757 A1 describes a catheter system and methods of medical uses of same, including diagnostic and treatment uses for the heart. U.S. Patent Pub. No. 2017/0319140 A1 is directed is directed to a catheter having a basket-shaped electrode assembly with a high electrode density. The basket-shaped electrode assembly may have a plurality of spines, such as up to twelve, each with a plurality of electrodes, such as up to sixteen. The distal ends of the plurality of spines are joined at a distal hub, all of which are fashioned from a single piece of superelastic material. International Patent Pub. No. WO 2020/251857 A1 describes an electrode basket having high-density circumferential band of electrodes. U.S. Patent Pub. No. 2002/0198522 A1 describes a medical device with three dimensional collapsible basket structure. Due to the small size of the spines and the electrodes, however, adhering the electrodes to the spines and then forming a spherical basket from the multiple linear spines can be a difficult task, increasing the manufacturing time and cost and the chances that the electrode fails due to an improper bond or misalignment. What is needed, therefore, are devices and methods of forming an improved basket assembly that can help to reduce the time required for manufacturing the basket assembly and alternative catheter geometries in general.

### SUMMARY OF THE INVENTION

The invention provides expandable basket assemblies for medical probes and methods of manufacture thereof, in accordance with the appended claims.

### SUMMARY OF THE DISCLOSURE

An expandable basket assembly for a medical probe may include a single unitary structure comprising a plurality of spines converging at a central spine intersection. The central spine intersection may be positioned on a longitudinal axis of the expandable basket assembly at a distal end thereof.

The single unitary structure may include a plurality of radial cutouts with each radial cutout defining an opening in each of the plurality of spines proximate the central spine intersection so that each opening extends for a length along each spine away from the central spine intersection.

Each cut-out may include a tadpole shaped cutout. The tadpole cutout may include a head portion contiguous to a circumference of a first virtual circle with a first radius r1 disposed about the central axis. The head portion may define a negative area approximating a second virtual circle with a second radius r2. The head portion may be connected to a slotted tail portion extending for a first length L1 along the spine and contiguous to an inside circumference of a third virtual circle having a third radius r3.

The first length of the slotted tail portion may be approximately 6-10 times that of the length of the radius r1 of the first virtual circle.

A center aperture may be disposed on the central spine intersection and the plurality of radial cutouts may be separated from the center aperture by a portion of the central spine intersection.

A cut-out may be disposed on the central axis to define a central negative area approximating a central circle including a central radius r0 smaller than the first radius r1.

The negative area of each of the tadpole cut-out may include approximately 0.2 mm-squared while the negative area of center aperture 212A may be approximately 0.05mm-squared so that the total negative area defined by all of the cut-outs may be approximately 1.4mm-squared.

A central void radius r0 may be approximately 0.13mm, the second radius r2 may be approximately 0.2mm, and the first radius r1 may be approximately 0.23mm.

The center aperture may include an area of about 0.01 mm-squared to about 0.4 mm-squared.

Each of the plurality of radial cutouts may include an area of about 0.1 mm-squared to about 0.55 mm-squared.

Each of the spines may include a first portion proximate to the central spine intersection with a first width, a second portion proximate the first portion with a second width less than the first portion, and a third portion proximate the second portion with a third width that is greater than the first width and greater than the second width.

Each radial cut-out may define a comet-shaped cutout with head portion with a slotted tapered tail extending to the proximal portion of each spine.

The first width may be about 0.15 mm to about 0.5 mm, the second width may be about 0.05 mm to about 0.35 mm, and the third width may be about 0.3 mm to about 0.7 mm.

The third portion of each spine may include an electrode attachment slot configured to accept an electrode. The electrode attachment slot may bisect each spine into two minor widths of about 0.05 mm to about 6 mm.

Each radial cut-out may include two teardrop cutouts attached at their narrow portions to define a single cutout on each spine of about 0.4 mm-squared.

The plurality of radial cutouts may extend from and are connected to the center aperture to form a single cutout.

The third portion of each spine may include a slot that bisects each spine into two minor widths each of which comprises a width of approximately 0.1 mm to approximately 6 mm.

The third portion of each spine may include a slot that bisects each spine into two minor portions with the slot comprising a width of approximately 0.05 mm to approximately 0.55 mm.

The center aperture may include a radius of approximately 0.4 mm to approximately 1.2 mm.

Each radial cutout may include an ellipse shape at an end furthest from the center aperture.

The ellipse shape may include a length of about 0.20 mm to about 0.55 mm and a width of about 0.1 mm to about 0.45 mm.

Each radial cutout may include a circular shape at an end furthest from the center aperture.

The circular shape may include a radius of approximately 0.05 mm to approximately 0.6 mm.

Each spine may include a thickness of about 0.03 mm to about 0.15 mm.

Each spine may include two connecting portions that connect to adjacent spines.

The connecting portions may include a width of about 0.12 mm to about 0.4 mm.

Two adjacent connecting portions may form a circular shape.

The circular shape may include a radius of approximately 0.25 mm to approximately 0.75 mm.

The plurality of spines may extend from the central spine intersection in an equiangular pattern such that respective angles between respectively adjacent spines are approximately equal.

The plurality of spines may include four to ten spines of the plurality of spines.

The plurality of spines may include six spines.

The plurality of spines may form an approximately spherical shape.

The plurality of spines may form an approximately oblate-spheroid shape.

The plurality of radial cutouts may include a centrosymmetric pattern.

The plurality of spines may include nitinol.

The plurality of spines may include cobalt chromium.

One or more electrodes may be coupled to each of the spines. Each electrode may define a lumen through the electrode so that a spine extends through the lumen of each of the one or more electrodes.

Each electrode may include a wire relief adjacent the lumen to allow for one or more wires to extend adjacent to the lumen.

The lumen may be disposed symmetrically about a longitudinal axis of the electrode.

The one or more electrodes may be configured to deliver electrical pulses for irreversible electroporation, the pulses having a peak voltage of at least 900 volts (V).

In an aspect, an expandable basket assembly for a medical probe may include a single unitary structure that may include a plurality of spines converging at a central spine intersection in a spiral pattern. The central spine intersection may be positioned on a longitudinal axis of the expandable basket assembly at a distal end thereof. Each of the plurality of spines may include a first width extending away from the distal end and a second width associated with the spiral pattern and narrower than the first width.

The spiral pattern may be logarithmic.

Each spine may include a pitch angle of approximately 60 degrees to approximately 105 degrees.

Each spine may include a pitch angle of approximately 100 degrees to approximately 140 degrees.

The central spine intersection may include a center aperture.

The center aperture may include a radius of approximately 0.01 mm to approximately 0.3 mm.

The first width may be approximately 0.1 mm to approximately 1.0 mm.

The second width may be approximately 0.05 mm to approximately 0.65 mm.

Each spine may include a tapering width disposed between the first and second widths.

One or more electrodes may be coupled to each of the spines. Each electrode may define a lumen through the electrode so that a spine extends through the lumen of each of the one or more electrodes.

Each electrode may include a wire relief adjacent the lumen to allow for one or more wires to extend adjacent to the lumen.

The lumen may be disposed symmetrically about a longitudinal axis of the electrode.

The one or more electrodes may be configured to deliver electrical pulses for irreversible electroporation, the pulses having a peak voltage of at least 900 volts (V).

In an aspect, an expandable basket assembly for a medical probe may include a single unitary structure that may include a plurality of spines converging at a central spine intersection. The central spine intersection may be positioned on a longitudinal axis of the expandable basket assembly at a distal end thereof. Each of the plurality of spines may include a first portion proximate the distal end, a second portion proximate the first portion, and a third portion proximate the second portion and wider than the first portion. The central spine intersection may include a center aperture.

The first portion may include a first width of approximately 0.05 mm to approximately 0.65 mm.

The third portion may include a third width of approximately 0.1 mm to approximately 1.0 mm.

The second portion may include a tapering width narrowing from the first portion to the third portion.

One or more electrodes may be coupled to each of the spines. Each electrode may define a lumen through the electrode so that a spine extends through the lumen of each of the one or more electrodes.

Each electrode may include a wire relief adjacent the lumen to allow for one or more wires to extend adjacent to the lumen.

The lumen is disposed symmetrically about a longitudinal axis of the electrode.

The one or more electrodes may be configured to deliver electrical pulses for irreversible electroporation, the pulses having a peak voltage of at least 900 volts (V).

In an aspect not defined by the appended claims, a method of constructing a medical probe may include cutting a planar sheet or tubular stock of material to form a plurality of spines having a central spine intersection and cutting a center aperture at the central spine intersection, cutting a plurality of radial cutouts with each radial cutout positioned on each spine of the plurality of spines, or cutting both the center aperture and the plurality of radial cutouts.

The center aperture may be cut to have an area of approximately 0.01 mm-squared to approximately 0.4 mm-squared.

The method also including cutting a plurality of radial cutouts with each radial cutout positioned on each spine of the plurality of spines.

Each of the plurality of radial cutouts may be cut to have an area of approximately 0.1 mm-squared to approximately 0.55 mm-squared.

Each of the spines may include a first portion distal to the central spine intersection with a first width, a second portion proximate the first portion with a second width less than the first width, and a third portion proximate the second portion and the central spine intersection with a third width that is less than the first width and greater than the second width.

The first width may be approximately 0.3 mm to approximately 0.7mm, the second width may be approximately 0.05 to approximately 0.35 mm, and the second width may be approximately 0.15 mm to approximately 0.5 mm.

The plurality of radial cutouts may extend from and are connected to the center aperture to form a single cutout.

Cutting the plurality of radial cutouts and cutting the center aperture may occur simultaneously.

The center aperture may include a radius of approximately 0.4 mm to approximately 1.2 mm.

Each radial cutout may be cut to form an ellipse shape at an end furthest from the center aperture.

The ellipse shape may include a length of approximately 0.20 mm to 0.55 mm and a width of approximately 0.1 mm to 0.45 mm.

Each spine may be cut to a thickness of approximately 0.03 mm to approximately 0.15 mm.

Cutting the plurality of spines may include cutting a connecting portion that connects to adjacent spines about a central spine intersection.

The connecting portion may be cut to a width of approximately 0.12 mm to approximately 0.4 mm.

Two adjacent connecting portions may be cut to form a circular shape.

The circular shape may be cut to have a radius of approximately 0.25 mm to approximately 0.75 mm.

The method may also include inserting each spine into a lumen of one or more electrodes and fitting ends of the plurality of spines to a tubular shaft sized to traverse vasculature such that the central spine intersection is positioned at a distal end of the medical probe and respective spines are movable from a tubular configuration to a bowed configuration.

Each electrode may include a relief adjacent the lumen to allow a wire to extend adjacent to the lumen.

The wire may be electrically insulated from the single spine.

The method may also include electrically connecting the wire to the one or more electrodes.

In aspects not defined by the appended claims, the plurality of spines may be cut from a tubular stock of material using one or more lasers.

### BRIEF DESCRIPTION OF THE DRAWINGS

FIG. 1 is a schematic pictorial illustration of a medical system including a medical probe whose distal end includes a basket assembly with electrodes, in accordance with an embodiment of the present invention;
FIG. 2A is a schematic pictorial illustration showing a perspective view of a medical probe in an expanded form, in accordance with an embodiment of the present invention;
FIG. 2B is a schematic pictorial illustration showing a side view of a medical probe in a collapsed form, in accordance with embodiments of the present invention;
FIG. 2C is a schematic pictorial illustration showing an exploded side view of a medical probe. The basket assembly of Fig. 2C is not defined by the present invention;
Fig. 2D is an exploded view of the contact force sensor 400 of Fig. 2C;
FIGS. 3A and 3B are schematic pictorial illustrations showing a profile outline of a basket assembly of a given medical device, in accordance with embodiments of the present invention;
FIG. 4 is a schematic pictorial illustration showing a side view of a plurality of linear spines forming a basket assembly, in accordance with an embodiment of the present invention;
FIGS. 5A and 5B are schematic pictorial illustrations of a method of forming a basket assembly, in accordance with an embodiment of the present invention. The basket assembly of Fig. 5B is not defined by the present invention;
FIG. 5C illustrates an embodiment where the proximal end of each spine is provided with a hole and reference notches to ensure correct alignment and retention of the spine to the irrigation tube, in accordance with an embodiment of the present invention;
FIG. 5D illustrates an embodiment that relies on a balloon to expand the spine assembly, in accordance with an embodiment of the present invention. The basket assembly of Fig. 5D is not defined by the present invention;
FIG. 5E illustrates a spine assembly formed by cutting a cylindrical tube stock with a laser. The basket assembly of Fig. 5E is not defined by the present invention;
FIG. 5F illustrates a spine assembly after shape setting of the spines in Fig. 5E into a spheroidal basket like shape;
FIG. 5G illustrates a spine assembly of FIG. 5F that relies on a balloon to expand the spine assembly;
FIGS. 6A, 6B, 6C, 6D, 6E, 6E1, 6E2, 6F, 6G, 6H, 6I, 6J, and 6K are schematic pictorial illustrations of central spine intersections. The basket assemblies of Figs. 6D, 6F and 6G are not defined by the present invention;
FIGS. 7A, 7B, 7C, 7D, 7E, 7F, 7G, 7H, 7I, and 7J are schematic pictorial illustrations showing a perspective view of various example electrodes;
FIGS. 8A and 8B are schematic pictorial illustrations showing various insulative jackets of a given medical device;
FIG. 9 is a schematic pictorial illustration of a method of cutting a plurality of linear spines from a planar sheet of material, in accordance with an embodiment of the present invention;
FIGS. 10A, 10B, 10C, and 10D are schematic pictorial illustrations of a method of cutting a plurality of linear spines from a planar sheet of material, in accordance with an embodiment of the present invention;
FIGS. 11A and 11B are schematic pictorial illustrations of a method of cutting a plurality of linear spines including one or more cutouts at a central spine intersection from a planar sheet of material, in accordance with an embodiment of the present invention; and
FIG. 12 is a flowchart illustrating a method of assembling a basket assembly, in accordance with an embodiment of the present invention.

### DETAILED DESCRIPTION

The following detailed description should be read with reference to the drawings, in which like elements in different drawings are identically numbered. The drawings, which are not necessarily to scale, depict selected embodiments and are not intended to limit the scope of the invention, which is defined by the appended claims. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%.

As used herein, the terms "patient," "host," "user," and "subject" refer to any human or animal subject and are not intended to limit the systems or methods to human use, although use of the subject invention in a human patient represents a preferred embodiment. In addition, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example. As well, the term "proximal" indicates a location closer to the operator or physician whereas "distal" indicates a location further away to the operator or physician.

As discussed herein, "operator" can include a doctor, surgeon, technician, scientist, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "bipolar" and "unipolar" when used to refer to ablation schemes describe ablation schemes which differ with respect to electrical current path and electric field distribution. "Bipolar" refers to ablation scheme utilizing a current path between two electrodes that are both positioned at a treatment site; current density and electric flux density is typically approximately equal at each of the two electrodes. "Unipolar" refers to ablation scheme utilizing a current path between two electrodes where one electrode including a high current density and high electric flux density is positioned at a treatment site, and a second electrode including comparatively lower current density and lower electric flux density is positioned remotely from the treatment site.

As discussed herein, the terms "biphasic pulse" and "monophasic pulse" refer to respective electrical signals. "Biphasic pulse" refers to an electrical signal including a positive-voltage phase pulse (referred to herein as "positive phase") and a negative-voltage phase pulse (referred to herein as "negative phase"). "Monophasic pulse" refers to an electrical signal including only a positive or only a negative phase. Preferably, a system providing the biphasic pulse is configured to prevent application of a direct current voltage (DC) to a patient. For instance, the average voltage of the biphasic pulse can be zero volts with respect to ground or other common reference voltage. Additionally, or alternatively, the system can include a capacitor or other protective component. Where voltage amplitude of the biphasic and/or monophasic pulse is described herein, it is understood that the expressed voltage amplitude is an absolute value of the approximate peak amplitude of each of the positive-voltage phase and/or the negative-voltage phase. Each phase of the biphasic and monophasic pulse preferably has a square shape including an essentially constant voltage amplitude during a majority of the phase duration. Phases of the biphasic pulse are separated in time by an interphase delay. The interphase delay duration is preferably less than or approximately equal to the duration of a phase of the biphasic pulse. The interphase delay duration is more preferably about 25% of the duration of the phase of the biphasic pulse.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer surface without departing from the scope of the present disclosure.

The term "temperature rating", as used herein, is defined as the maximum continuous temperature that a component can withstand during its lifetime without causing thermal damage, such as melting or thermal degradation (e.g., charring and crumbling) of the component.

The present disclosure is related to systems, methods or uses and devices which utilize end effectors including electrodes affixed to spines. Example systems, methods, and devices of the present disclosure may be particularly suited for IRE ablation of cardiac tissue to treat cardiac arrhythmias. Ablative energies are typically provided to cardiac tissue by a tip portion of a catheter which can deliver ablative energy alongside the tissue to be ablated. Some example catheters include three-dimensional structures at the tip portion and are configured to administer ablative energy from various electrodes positioned on the three-dimensional structures. Ablative procedures incorporating such example catheters can be visualized using fluoroscopy.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation.

RF approaches can have risks that can lead to tissue charring, burning, steam pop, phrenic nerve palsy, pulmonary vein stenosis, and esophageal fistula. Cryoablation is an alternative approach to RF ablation that can reduce some thermal risks associated with RF ablation. However maneuvering cryoablation devices and selectively applying cryoablation is generally more challenging compared to RF ablation; therefore, cryoablation is not viable in certain anatomical geometries which may be reached by electrical ablation devices.

IRE as discussed in this disclosure is a non-thermal cell death technology that can be used for ablation of atrial arrhythmias. To ablate using IRE/PEF, biphasic voltage pulses are applied to disrupt cellular structures of myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

Electroporation can be induced by applying a pulsed electric field across biological cells to cause reversable (temporary) or irreversible (permanent) creation of pores in the cell membrane. The cells have a transmembrane electrostatic potential that is increased above a resting potential upon application of the pulsed electric field. While the transmembrane electrostatic potential remains below a threshold potential, the electroporation is reversable, meaning the pores can close when the applied pulse electric field is removed, and the cells can self-repair and survive. If the transmembrane electrostatic potential increases beyond the threshold potential, the electroporation is irreversible, and the cells become permanently permeable. As a result, the cells die due to a loss of homeostasis and typically die by programmed cell death or apoptosis, which is believed to leave less scar tissue as compared to other ablation modalities. Generally, cells of differing types have differing threshold potential. For instance, heart cells have a threshold potential of approximately 500 V/cm, whereas for bone it is 3000 V/cm. These differences in threshold potential allow IRE to selectively target tissue based on threshold potential.

The solution of this disclosure includes systems and methods for applying electrical signals from catheter electrodes positioned in the vicinity of myocardial tissue, preferably by applying a pulsed electric field effective to induce electroporation in the myocardial tissue. The systems and methods can be effective to ablate targeted tissue by inducing irreversible electroporation. In some examples, the systems and methods can be effective to induce reversible electroporation as part of a diagnostic procedure. Reversible electroporation occurs when the electricity applied with the electrodes is below the electric field threshold of the target tissue allowing cells to repair. Reversible electroporation does not kill the cells but allows a physician to see the effect of reversible electroporation on electrical activation signals in the vicinity of the target location. Example systems and methods for reversible electroporation is disclosed in U.S. Patent Publication 2021/0162210.

The pulsed electric field, and its effectiveness to induce reversible and/or irreversible electroporation, can be affected by physical parameters of the system and biphasic pulse parameters of the electrical signal. Physical parameters can include electrode contact area, electrode spacing, electrode geometry, etc. examples presented herein generally include physical parameters adapted to effectively induce reversible and/or irreversible electroporation. Biphasic pulse parameters of the electrical signal can include voltage amplitude, pulse duration, pulse interphase delay, inter-pulse delay, total application time, delivered energy, etc. In some examples, parameters of the electrical signal can be adjusted to induce both reversible and irreversible electroporation given the same physical parameters. Examples of various systems and methods of ablation including IRE are presented in U.S. Patent Publications 2021/0169550A1, 2021/0169567A1, 2021/0169568A1, 2021/0161592A1, 2021/0196372A1, 2021/0177503A1, 2021/0186604A1, 2021/10162210, and 2021/0077180.

To deliver pulsed field ablation (PFA) in an IRE (irreversible electroporation) procedure, electrodes should contact the tissue being ablated with a sufficiently large surface area. As described hereinbelow, the medical probe includes a tubular shaft including proximal and distal ends, and a basket assembly at the distal end of the tubular shaft. The basket assembly includes a single unitary structure. The unitary structure can include a plurality of linear spines formed from a planar sheet of material and one or more electrodes coupled to each of the spines. The plurality of linear spines can converge at a central spine intersection including one or more cutouts. The cutouts can allow for bending of each spine such that the spines form an approximately spherical or oblate-spheroid basket assembly. It is noted that the cutouts (in various configurations described and illustrated in the specification) allows the basket to be compressed into a much smaller form factor when undeployed (or undergoing a retraction into a delivery sheath) without buckling or plastic deformation.

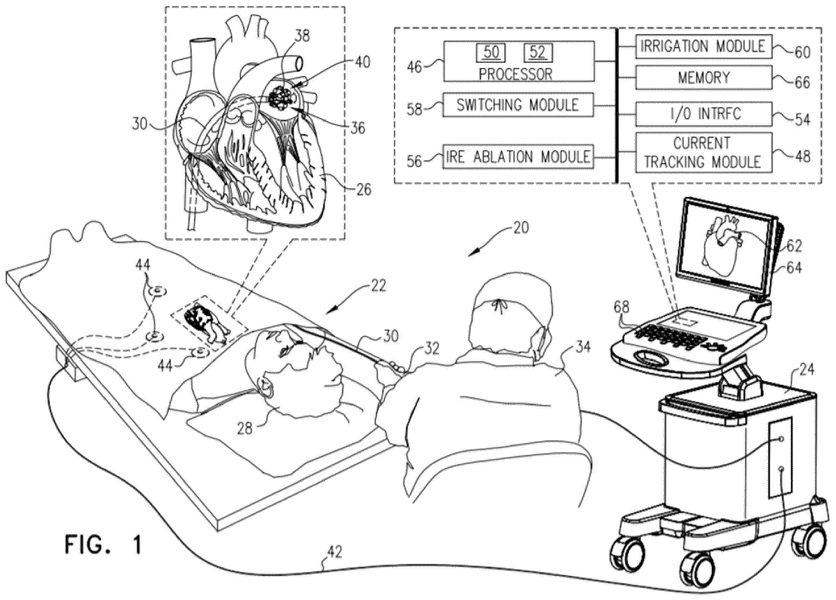

FIG. 1 is a schematic, pictorial illustration of a medical system 20 including a medical probe 22 and a control console 24, in accordance with an embodiment of the present invention. Medical system 20 may be based, for example, on the CARTO^{®} system, produced by Biosense Webster Inc. of 31 Technology Drive, Suite 200, Irvine, CA 92618 USA. In embodiments described hereinbelow, medical probe 22 can be used for diagnostic or therapeutic treatment, such as for performing ablation procedures in a heart 26 of a patient 28. Alternatively, medical probe 22 may be used, mutatis mutandis, for other therapeutic and/or diagnostic purposes in the heart or in other body organs.

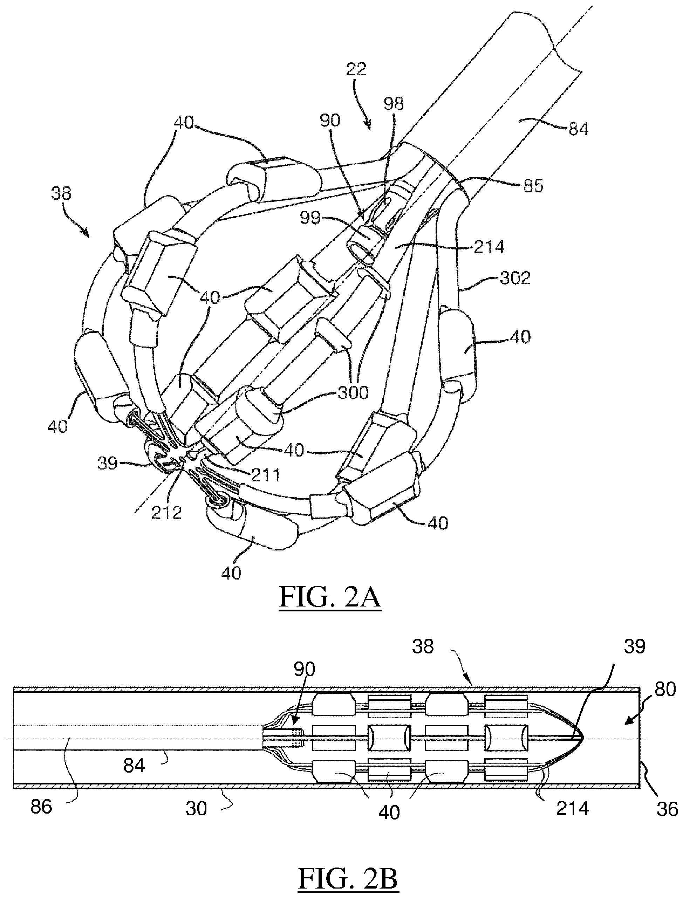

Medical probe 22 includes a flexible insertion tube 30 and a handle 32 coupled to a proximal end of the tubular shaft. During a medical procedure, a medical professional 34 can insert probe 22 through the vascular system of patient 28 so that a distal end 36 of the medical probe enters a body cavity such as a chamber of heart 26. Upon distal end 36 entering the chamber of heart 26, medical professional 34 can deploy a basket assembly 38 approximate a distal end 36 of the medical probe 22. Basket assembly 38 can include a plurality of electrodes 40 affixed to a plurality of spines 214, as described in the description referencing FIGS. 2A and 2B hereinbelow. To start performing a medical procedure such as irreversible electroporation (IRE) ablation, medical professional 34 can manipulate handle 32 to position distal end 36 so that electrodes 40 engage cardiac tissue at a desired location or locations. Upon positioning the distal end 36 so that electrodes 40 engages cardiac tissue, the medical professional 34 can activate the medical probe 22 such that electrical pulses are delivered by the electrodes 40 to perform the IRE ablation.

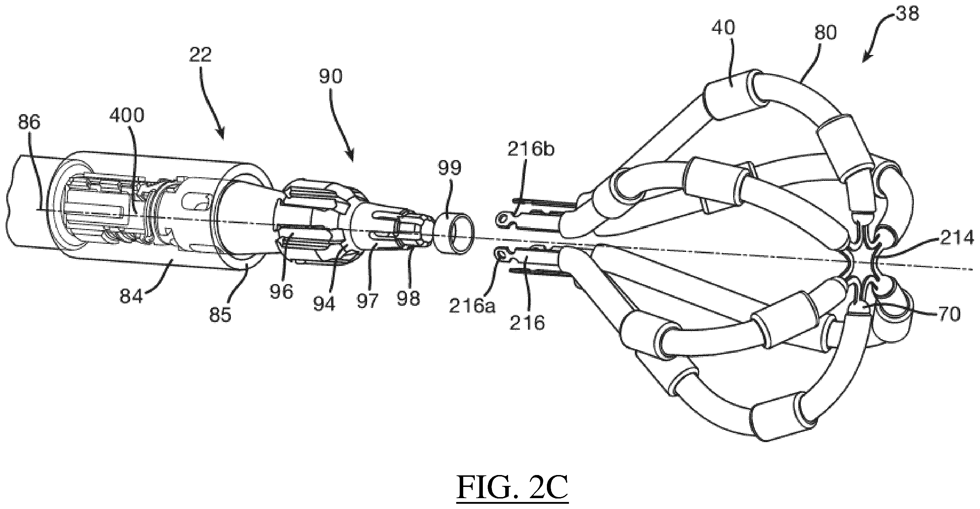

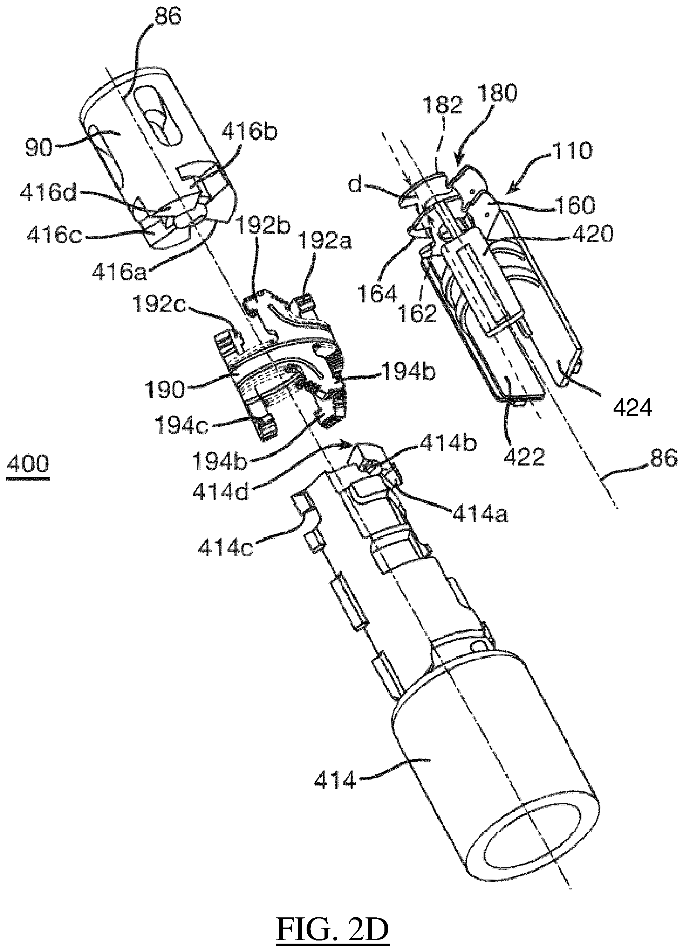

The medical probe 22 can include a guide sheath and a therapeutic catheter, wherein the guide sheath includes the flexible insertion tube 30 and the handle 32 and the therapeutic catheter includes the basket assembly 38, electrodes 40, and a tubular shaft 84 (see FIGS. 2 through 4). The therapeutic catheter is translated through the guide sheath so that the basket assembly 38 is positioned in the heart 26. The distal end 36 of the medical probe 22 corresponds to a distal end of the guide sheath when the basket assembly 38 is contained within the flexible insertion tube 30, and the distal end 36 of the medical probe 22 corresponds to a distal end of the basket assembly 38 when the basket assembly 38 is extended from the distal end of the guide sheath. The medical probe 22 can be alternatively configured to include a second handle on the therapeutic catheter and other features as understood by a person skilled in the pertinent art.

In the configuration shown in FIG. 1, control console 24 is connected, by a cable 42, to body surface electrodes, which typically include adhesive skin patches 44 that are affixed to patient 28. Control console 24 includes a processor 46 that, in conjunction with a tracking module 48, determines location coordinates of distal end 36 inside heart 26. Location coordinates can be determined based on electromagnetic position sensor output signals provided from the distal portion of the catheter when in the presence of a generated magnetic field. Location coordinates can additionally, or alternatively be based on impedances and/or currents measured between adhesive skin patches 44 and electrodes 40 that are affixed to basket assembly 38. In addition to being used as location sensors during a medical procedure, electrodes 40 may perform other tasks such as ablating tissue in the heart.

As described hereinabove, in conjunction with tracking module 48, processor 46 may determine location coordinates of distal end 36 inside heart 26 based on impedances and/or currents measured between adhesive skin patches 44 and electrodes 40. Such a determination is typically after a calibration process relating the impedances or currents to known locations of the distal end has been performed. While embodiments presented herein describe electrodes 40 that are preferably configured to deliver IRE ablation energy to tissue in heart 26, configuring electrodes 40 to deliver any other type of ablation energy to tissue in any body cavity is considered to be within the scope of the present invention. Furthermore, although described in the context of being electrodes 40 that are configured to deliver IRE ablation energy to tissue in the heart 26, one skilled in the art will appreciate that the disclosed technology can be applicable to electrodes used for mapping and/or determining various characteristics of an organ or other part of the patient's 28 body.

Processor 46 may include real-time noise reduction circuitry 50 typically configured as a field programmable gate array (FPGA), followed by an analog-to-digital (A/D) signal conversion integrated circuit 52. The processor can be programmed to perform one or more algorithms and uses circuitry 50 and circuit 52 as well as features of modules to enable the medical professional 34 to perform the IRE ablation procedure.

Control console 24 also includes an input/output (I/O) communications interface 54 that enables control console 24 to transfer signals from, and/or transfer signals to electrodes 40 and adhesive skin patches 44. In the configuration shown in FIG. 1, control console 24 additionally includes an IRE ablation module 56 and a switching module 58.

IRE ablation module 56 is configured to generate IRE pulses including peak power in the range of tens of kilowatts. In some examples, the electrodes 40 are configured to deliver electrical pulses including a peak voltage of at least 900 volts (V). The medical system 20 performs IRE ablation by delivering IRE pulses to electrodes 40. Preferably, the medical system 20 delivers biphasic pulses between electrodes 40 on the spine. Additionally, or alternatively, the medical system 20 delivers monophasic pulses between at least one of the electrodes 40 and a skin patch.

In order to dissipate the heat and to improve the efficiency of the ablation process, system 20 supplies irrigation fluid (e.g., a saline solution) to distal end 36 and to the electrodes 40 via a channel (not shown) in tubular shaft 84 (see FIGS. 2A through 2C). Additionally, or alternatively, irrigation fluid can be supplied through the flexible insertion tube 30. Control console 24 includes an irrigation module 60 to monitor and control irrigation parameters, such as the pressure and the temperature of the irrigation fluid. It is noted that while the preference for the exemplary embodiments of the medical probe is for IRE or PFA, the medical probe may also be used separately only for RF ablation (unipolar mode with an external grounding electrode or bipolar mode) or in combination with IRE and RF ablations sequentially (certain electrodes in IRE mode and other electrodes in RF mode) or simultaneously (groups of electrodes in IRE mode and other electrodes in RF mode).

Based on signals received from electrodes 40 and/or adhesive skin patches 44, processor 46 can generate an electroanatomical map 62 that shows the location of distal end 36 in the patient's body. During the procedure, processor 46 can present map 62 to medical professional 34 on a display 64, and store data representing the electroanatomical map in a memory 66. Memory 66 may include any suitable volatile and/or non-volatile memory, such as random-access memory or a hard disk drive.

In some embodiments, medical professional 34 can manipulate map 62 using one or more input devices 68. In alternative embodiments, display 64 may include a touchscreen that can be configured to accept inputs from medical professional 34, in addition to presenting map 62.

FIG. 2A is a schematic pictorial illustration showing a perspective view of a medical probe 22 including a basket assembly 38 in an expanded form when unconstrained, such as by being advanced out of an insertion tube lumen 80 (FIG. 2B) at a distal end 36 of an insertion tube 30 (FIG. 2B). The medical probe 22 illustrated in FIG. 2A lacks the guide sheath illustrated in FIG. 1. FIG. 2B shows the basket assembly in a collapsed form within insertion tube 30 of the guide sheath. In the expanded form (FIG. 2A), spines 214 bow radially outwardly and in the collapsed form (FIG. 2B) the spines are arranged generally along a longitudinal axis 86 of insertion tube 30.

As shown in FIG. 2A, basket assembly 38 includes a plurality of flexible spines 214 that are formed at the end of a tubular shaft 84 and are connected at both ends. During a medical procedure, medical professional 34 can deploy basket assembly 38 by extending tubular shaft 84 from insertion tube 30 causing basket assembly 38 to exit insertion tube 30 and transition to the expanded form. Spines 214 may have elliptical (e.g., circular) or rectangular (that may appear to be flat) cross-sections, and include a flexible, resilient material (e.g., a shape-memory alloy such as nickel-titanium, also known as Nitinol) forming a strut as will be described in greater detail herein.

As shown in FIG. 2A, the plurality of flexible linear spines 214 converge at a central spine intersection 211. In some examples central spine intersection 211 can include one or more cutouts 212 that allow for bending of the spines 214 when each spine respective attachment end 216 (FIG. 2C) is connected to the spine retention hub 90 which may include a flow diverter for irrigation fluid, described in more detail below.

In embodiments described herein, one or more electrodes 40 positioned on spines 114 of basket assembly 38 can be configured to deliver ablation energy (RF and/or IRE) to tissue in heart 26. Additionally, or alternatively, the electrodes can also be used to determine the location of basket assembly 38 and/or to measure a physiological property such as local surface electrical potentials at respective locations on tissue in heart 26. The electrodes 40 can be biased such that a greater portion of the one or more electrodes 40 face outwardly from basket assembly 38 such that the one or more electrodes 40 deliver a greater amount of electrical energy outwardly away from the basket assembly 38 (i.e., toward the heart 26 tissue) than inwardly. Electrodes 40 are coupled to the spine 214 via electrode inserts 300 that can be bonded or fused to spines 214 while holding electrodes 40 in a fixed position on the spine 214.

As shown in Fig. 2A, a spine 214 is shown with one electrode 40 hidden so that electrode inserts can be seen. Inserts 300 can be made from an insulative material to insulate or isolate electrode 40 from contact or conduction with spine 214. The material for insert 300 can be any suitable insulative biocompatible material as long as such material can withstand at least 1900 Volts with at least 20 amperes of current. The spines may also be provided with an insulative cover 302 of a biocompatible polymer such as, for example, polyurethane to further insulate or isolate spines 214 from contact with body tissues or fluids. The insert holder 300 can be bonded directly to the 302 or spine 214 and subsequently further bonded to the insulative spine covers 302.

Examples of materials ideally suited for forming electrodes 40 include gold, platinum and palladium (and their respective alloys). These materials also have high thermal conductivity which allows the minimal heat generated on the tissue (i.e., by the ablation energy delivered to the tissue) to be conducted through the electrodes to the back side of the electrodes (i.e., the portions of the electrodes on the inner sides of the spines), and then to the blood pool in heart 26.

Basket assembly 38 has a distal end 39. The medical probe 22 can include a spine retention hub 90 that extends longitudinally from a distal end of tubular shaft 84 towards distal end 39 of basket assembly 38. As described supra, control console 24 includes irrigation module 60 that delivers irrigation fluid to basket assembly 38 through tubular shaft 84.

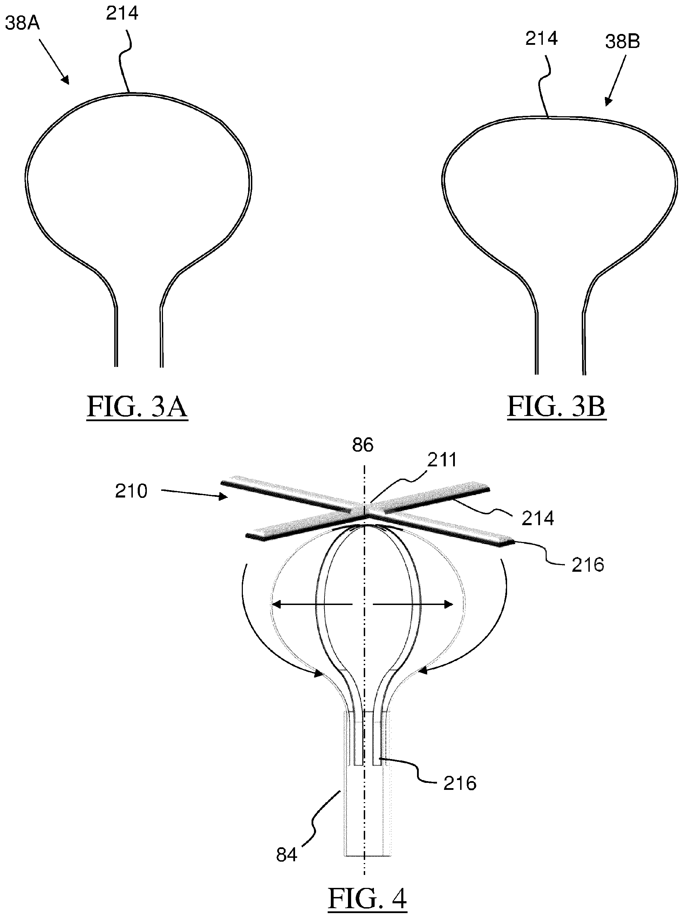

Turning to FIG. 2C, basket assembly 38 includes a single unitary structure that includes a plurality of linear spines 214 formed from a planar sheet of material 910 (shown more clearly in FIGS. 3 and 4A). The spines 214 may be concealed and covered by at least one or more extrusion layers 80, which electrode 40 are disposed around. The one or more extrusion layer 70, 80 may include two halves connect to each other and enable be placed around each spin 214. In other embodiments, the one or more extrusion layers 80 may be overmolded on each spine 214 or over end portions of each spine 214 as is the case for extrusion layers 70. The spine retention hub 90 can be inserted into the tubular shaft 84 and attached to the tubular shaft 84. Spine retention hub 90 can include a cylindrical member 94 including a plurality of relief lands 96, an upper portion 97, and multiple irrigation openings 98 positioned about the upper portion 97, and at least one spine retention hub electrode 99, or some combination thereof. Relief lands 96 can be disposed on the outer surface of cylindrical member 94 and configured to allow a portion of each spine 214, such as each spine attachment end 216, to be fitted into a respective relief land 96 and attached/locked via an attachment end hole 216a and an attachment end neck 216b. The attachment end 216 can be a generally linear end of the spine 214. The attachment end 216 can be configured to extend outwardly from the spine retention hub 90 such that the basket assembly 38 is positioned outwardly from the spine retention hub 90 and, consequently, outwardly from the tubular shaft 84. In this way, the spine 214 can be configured to position the basket assembly 38 distally from the distal end of the tubular shaft 84 and distal from the distal end of the insertion tube 30 when the basket assembly is deployed.

As described supra, control console 24 includes irrigation module 60 that delivers irrigation fluid to distal end 36. The multiple irrigation openings 98 can be angled to spray or otherwise disperse of the irrigation fluid to either a given electrode 40 or to tissue in heart 26. Since electrodes 40 do not include irrigation openings that deliver irrigation fluid, the configuration described hereinabove enables heat to be transferred from the tissue (i.e., during an ablation procedure) to the portion of the electrodes on the inner side of the spines 214, and the electrodes 40 can be cooled by aiming the irrigation fluid, via irrigation openings 98, at the portion of the electrodes 40 on the inner side of the spines 214. Spine retention hub electrode (usable as a reference electrode) 99 disposed at a distal end of retention hub 90 can be used in combination with electrodes 40 on the spines 214, or alternatively, can be used independently from electrodes 40 for reference mapping or ablation.

Fig. 2D is an exploded view for contact force sensor 400 referenced in Fig. 2C. As shown in Fig. 2D, the contact force sensor 400 is disposed inside tube 84 and proximally in relation to the basket assembly 38 and as close as possible to the basket 38 so that contact with cardiac tissue by the spines 214 can be transmitted to the contact force sensor 400. Contact force sensor 400 includes coupler 414 provided with a plurality of notches 414a, 414b, 414c on the periphery of the cylindrical member or coupler 414 for corresponding engagement with protrusions 194a, 194b, 194c of beam coupling member 190. A spine retention hub or coupler 96 is provided with notches 416a, 416b, 416c that mates with protrusions 192a, 192b, 192c of beam coupling member 190. Flat surfaces 416d (angled with respect to axis 86 for spine retention hub or coupler 96) are formed whereby each flat surface 416d is angulated with respect to the axis 86 so that each flat surface is complementary to the angulation 190 defined by the helicoid path of ramp 194a, 194b, 194c (i.e., helix angle). Three flat surfaces (not shown due to the perspective view) 414d are also provided for coupler 414 in a configuration similar to flat surface 416d of spine retention hub or coupler 96 in that the three flat surfaces 414d are also angulated with respect to the axis 86 so that each flat surface 414d of coupler 414 are generally parallel to the angulation path 190 defined by the helicoid ramp 194a, 194b, 194c as well as flat surface 416d.

The location sensor coils 422 and 424 are mounted to a coupler 414 (for coupling with hub 96) in a generally equiangular configuration about the axis 86. Coupler 414 is disposed inside of tubular shaft 84 towards a proximal portion of the catheter. It is noted that while two coils (for X and Y axes in the cartesian XYZ coordinate) are used in an exemplary embodiment to determine the location of these coils (as mounted to the coupler 414 and thereby the location of the basket spines as the distance between basket spines and the location sensor is known), in certain circumstances, only one location sensing coil may be utilized if the other two axes are known via other visualization techniques. As well, three location sensing coils may also be used depending on the packaging constraints of the catheter.

Each of the trefoil force sensor segment 160, 162, 164 for flex circuit 110 is mounted in the beam coupling member 190 such that each segment 160, 162, 164 has a counterpart segment with flex circuit 180. For example, segment 162 of flex circuit 110 is mounted to be parallel to segment 182 of flex circuit 180 at a specified distance "d" (which distance "d" can change when forces are applied to coupler 90 or 414). The remainder of the force sensor coil segments 162 and 164 of flex circuit 110 are mounted in a similar manner with the respective trefoil force sensor segment of flex circuit 180. Displacement for each pair of trefoil force sensor segment will allow console 24 to determine the angle and direction of forces being applied to which one of the pie-shaped force sensor coil segment pairs. For example, when distance "d" (opposite facing arrows in FIG. 2D) between force sensor coil segments 162 and 182 is changed without the distance on the other two pair of force sensor coil segments being changed, then the processor of the system is able to determine that a force is being applied along one of the directions designated by the dual-facing arrow (FIG. 2D).

Details of the contact force sensor are provided in US Patent Application Publication No. US20210077180A1 published March 18, 2021.

FIGS. 3A and 3B are schematic pictorial illustrations showing a profile outline of a basket assembly 38A, 38B such that when the basket assembly is deployed the spines define a three-dimensional shape including the profile. The basket assembly can be approximately spheroid including an approximately circular profile as shown in FIG. 3A. The basket assembly can have an approximately oblate-spheroid shape including an approximately elliptical profile as shown in FIG. 3B. Although not every variation of shape is shown or described herein, one skilled in the art will appreciate that spines 214 can be further configured to form other various shapes as would be suitable for the particular application.

By including spines 214 configured to form various shapes when in the expanded form, basket assembly 38 can be configured to position the various electrodes 40 attached to spines 214 at various locations, with each location being nearer or farther from the distal end of tubular shaft 84. For example, electrode 40 attached to spine 214 illustrated in FIG. 3A near the middle of spine 214 would be farther from the distal end of tubular shaft 84 than spine 214 illustrated in FIG. 3B when basket assembly 38 is in the expanded form. In addition, each spine 214 may have an elliptical (e.g., circular) or rectangular (that may appear to be flat) cross-section, and include a flexible, resilient material (e.g., a shape-memory alloy such as nickel-titanium (also known as Nitinol), cobalt chromium, or any other suitable material).

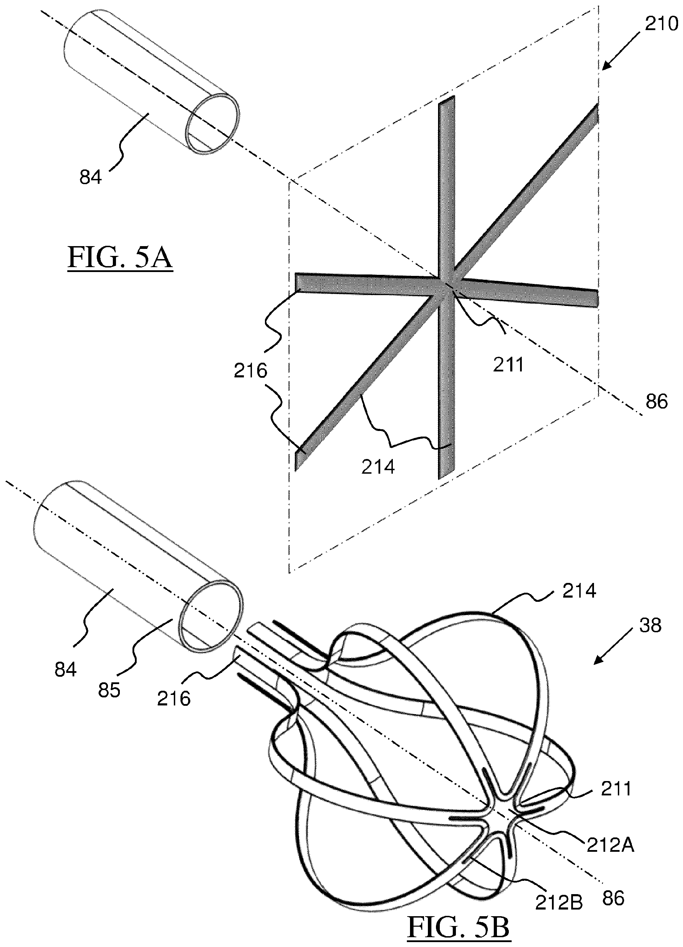

FIGS. 4, 5A and 5B are schematic pictorial illustrations showing views of spines 214 forming basket assembly 38. FIG. 4 provides one example of how planar sheet of material 910 may be assembled together with tubular shaft 84 whereby each spine 214 bends or curves when respective attachment ends 216 are connected to spine retention hub 90. As shown in FIG. 5A, the spines 214 can be formed from a single sheet of planar material 910 to form a generally star shape. In other words, spines 214 can be formed from the single sheet of planar material such that the spines 214 converge toward a central spine intersection 211. The intersection 211 can be a solid piece of material (as shown in FIG. 5A) or include one or more cutouts 212 (as shown in FIG. 5B). Basket assembly 38 can include a number of spines 214 ranging from about four to about ten spines from a single sheet of planar material 910.

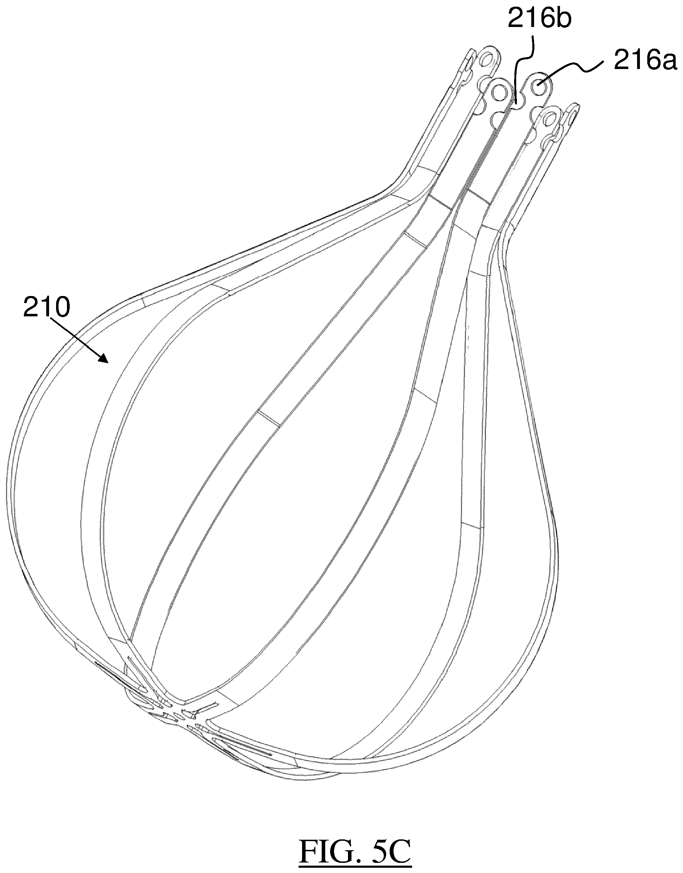

The spine assembly 210 can be physically connected to the tubular member 84 via a suitable technique such as adhesive or molding. In one embodiment shown here in Fig. 5C, eyelet 216a as well as locators 216b can be provided to aid in assembly as well as physical retention of the spines to the tubular member 84.

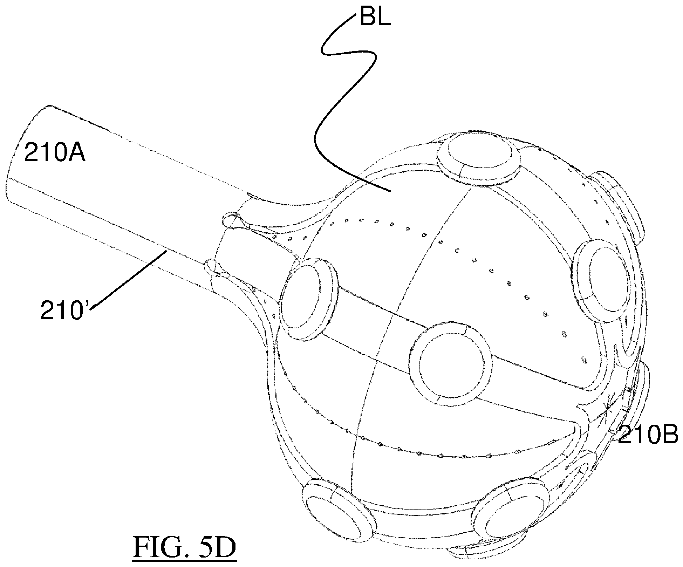

Where it is desired, a balloon BL can be provided as shown in Fig. 5D inside the spine assembly 210' to ensure full expansion of the spine assembly 210' from a cylindrical form factor into a spheroidal form as shown in Fig. 5C.

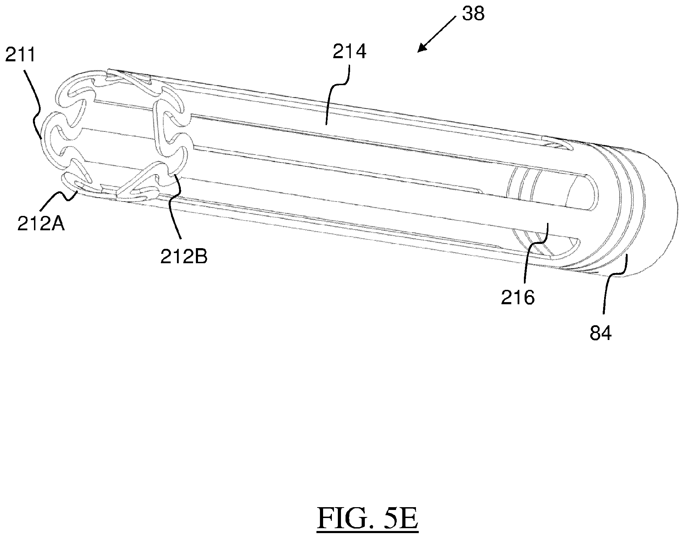

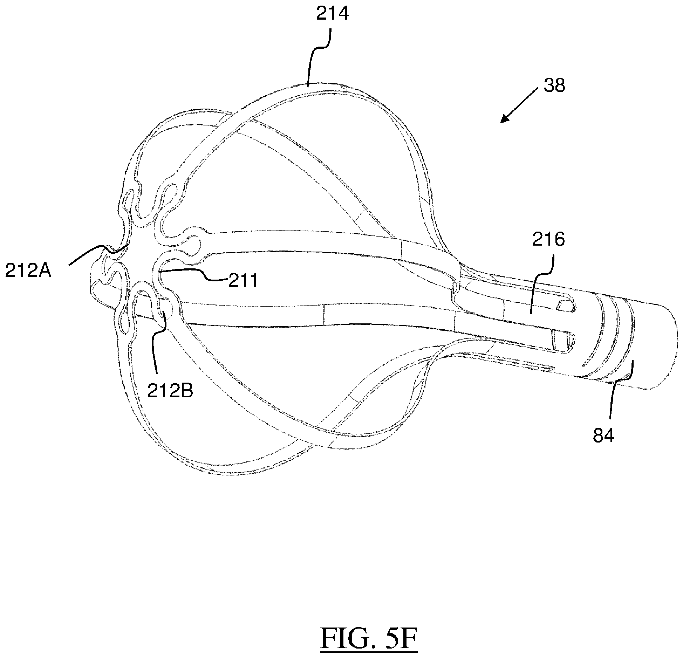

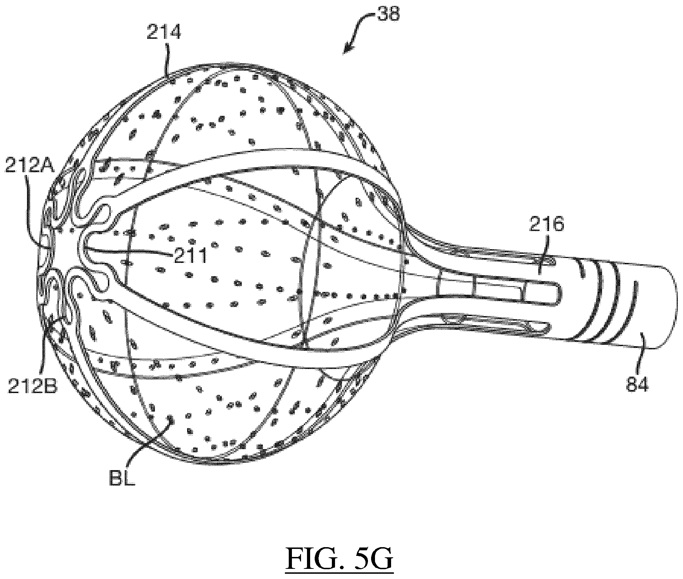

The spine assembly can be made from a tubular cylindrical stock material so that the proximal portion 210A and distal portion 210B are of one-piece material. The tubular stock is cut into a desired shape for the spine assembly 210' as shown in Fig. 5E. Thereafter, the cut tube can be shape set (or heat set) as is known by those skilled in the art to provide for the spheroidal spine configuration shown in Fig. 5F. When cut from tubular cylindrical stock material, the expandable basket assembly 38 may be also include a tubular shaft 84 which is formed with the spines 214 as shown in FIG. 5F and do not have to be attached since they are connected as one piece. Much like FIG. 5D an expandable basket assembly 38 formed from tubular cylindrical stock material may retain a balloon BL to ensure full expansion of the spine assembly 38 as shown in FIG. 5G.

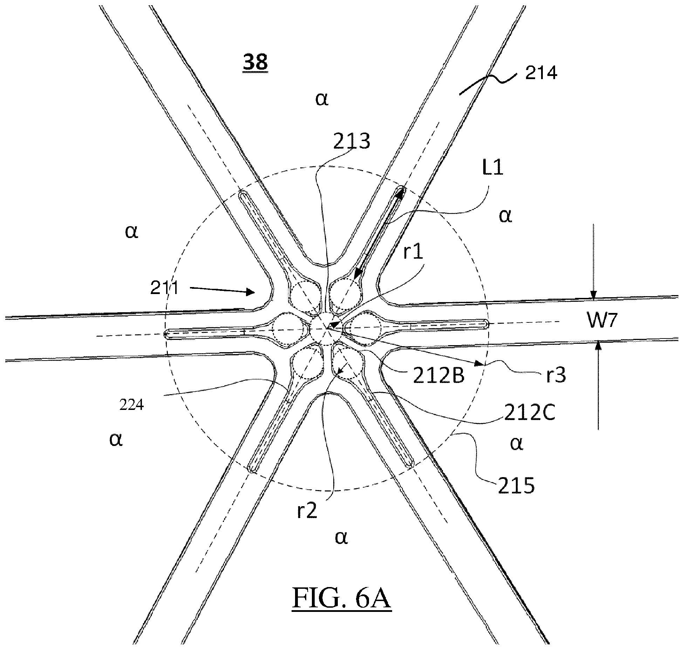

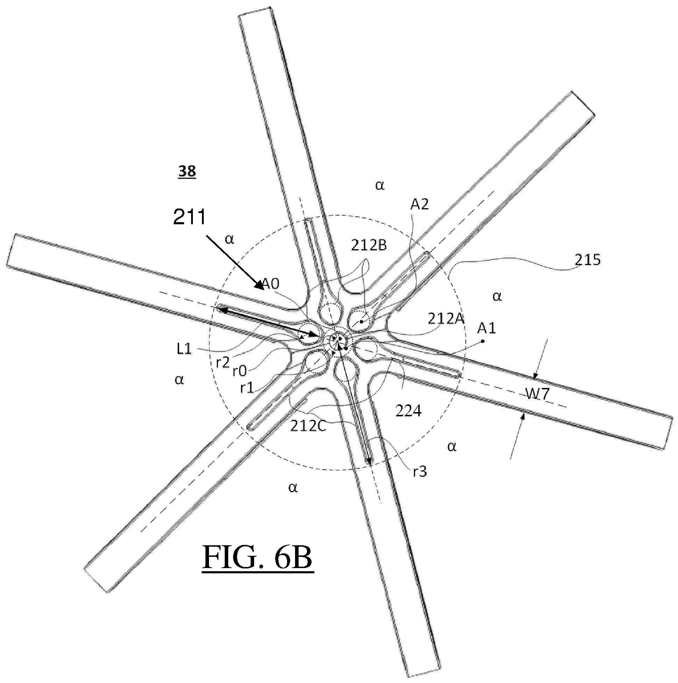

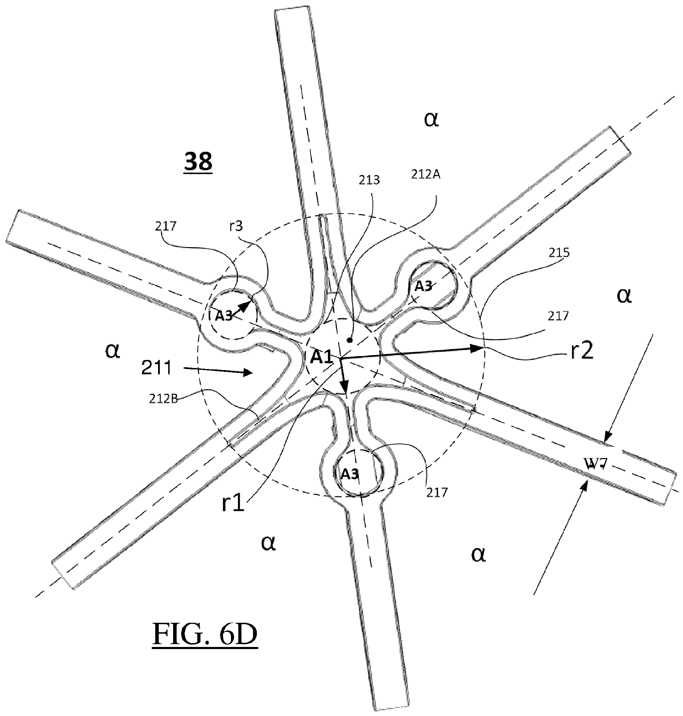

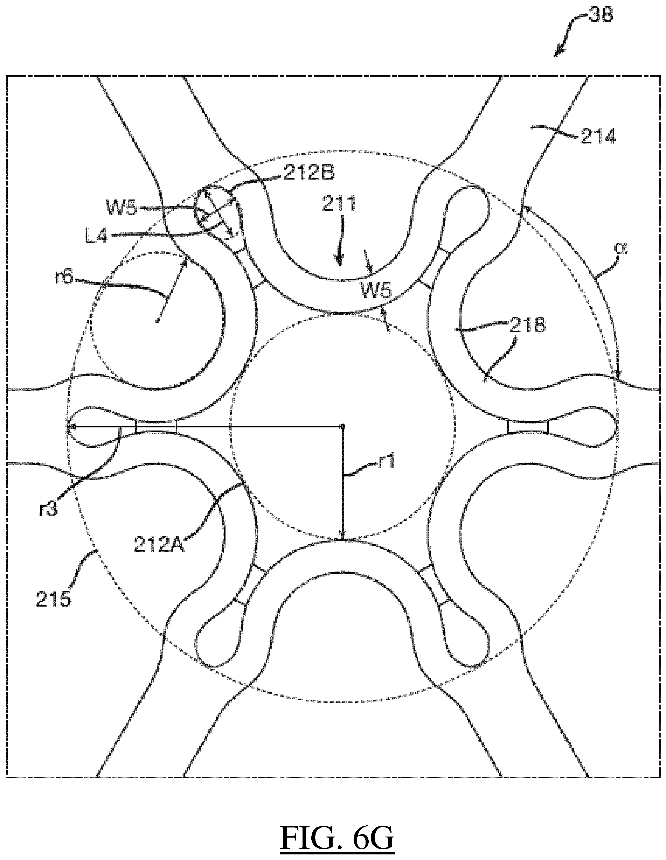

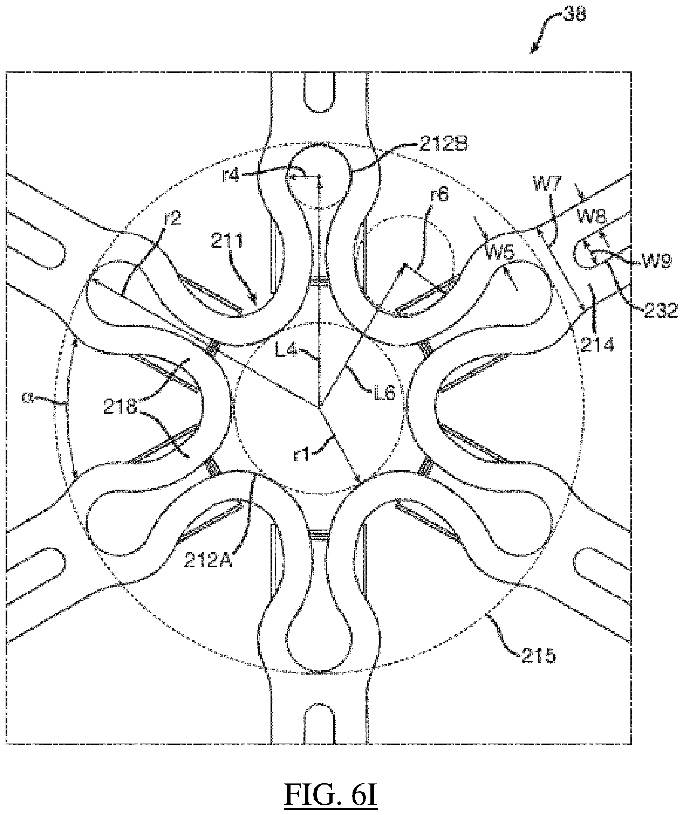

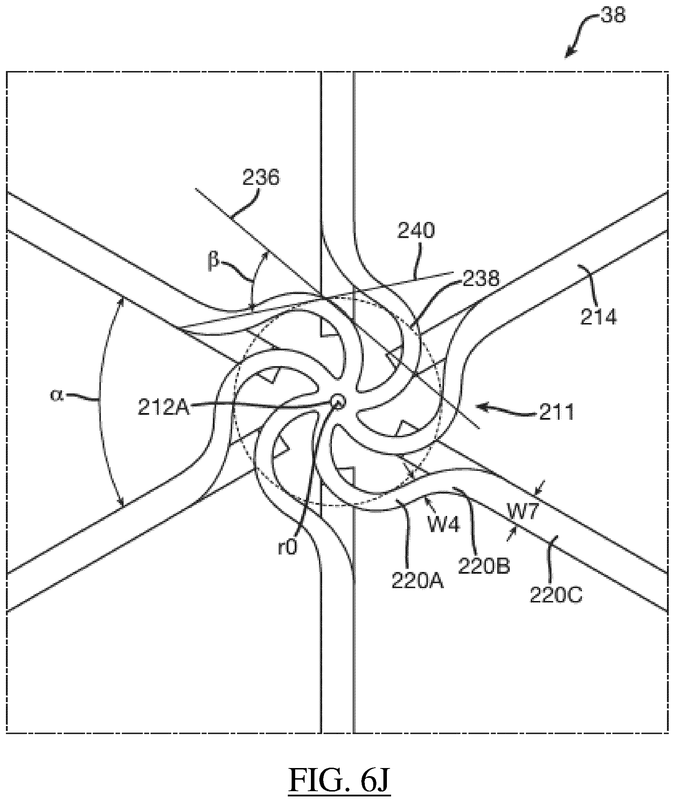

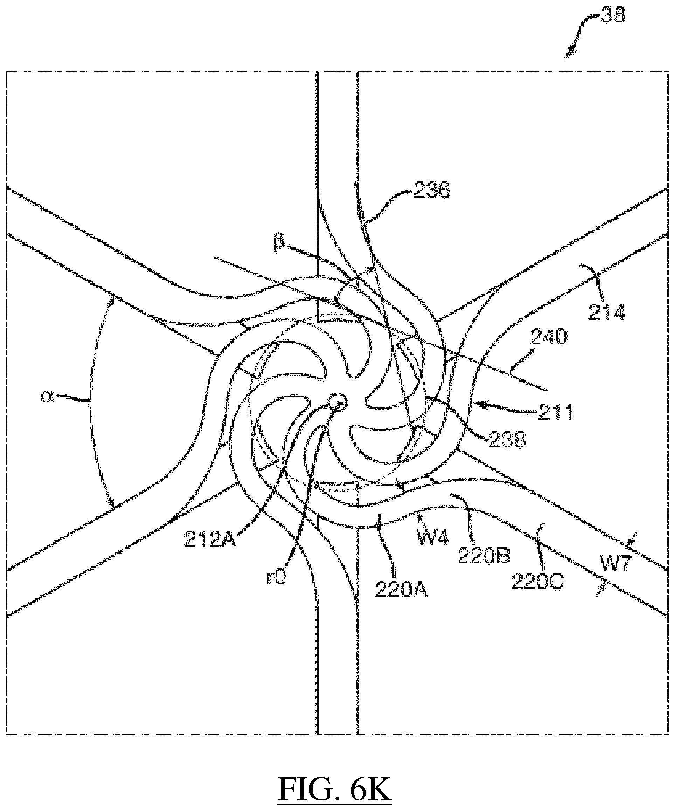

FIGS. 6A through 6K are schematic pictorial illustrations of top-down views of expandable basket assembly 38, showing various examples of one or more cutouts 212 on central spine intersection 211. As shown, intersection 211 can include a single discrete cutout as shown, for example in FIGS. 6D, 6G, and 6I that includes a central aperture 212A connected to radial cutouts 212B. Alternatively, intersection 211 can include two or more radial cutouts 212B, 212C with or without a central aperture cutout 212A as provided as an example in FIGS. 6A and 6B. The one or more cutouts 212A, 212B, 212C can include a variety of patterns, such as centrosymmetric (i.e., symmetric with respect to a central point), and equiangular (i.e., including equal angles) to allow for equal bending among the spines 214 as well as disproportional and asymmetric to allow for unequal bending of spines 214 to alter structural stability. In certain instances, when basket assembly 38 includes an even number of spines 214, the pattern of the one or more cutouts 212 can alter between every other spine, as illustrated in FIG. 6D. In some examples, one or more cutouts 212 can extend along a portion of each spine 214. In other examples, such as FIGS. 6J and 6K, the spines 214 may form a spiral pattern about the central spine intersection 211. Each of the designs illustrated in FIGS. 6A-6K will be discussed separately.

In FIG. 6A, the center (on axis 86) of the radiating spines 214 for basket 38 does not have a cut-out so that there is no void at the center of the basket to act as sharp edge surface (at the edge of such center aperture) against biological tissues. To allow for consistent folding of the spines near the distal portion of basket 38, each spine is provided with a tadpole shaped cutout 224 that extends from the head portion 212B to tail portion 212C. The head portions 212B are arrayed so that the head portions 212B are contiguous to an outside circumference of first virtual circle 213 with radius r1. Each head portion 212B has a negative surface area that can be approximated by a second virtual circle with radius r2 of approximately 90% of the first radius r1. The tail portions 212C are bounded by a third virtual circle 215 with a radius r3 approximately 10 times that of the first radius. The length L1 of each of the tail portion includes approximately 1.5 times that of the width W7 of the spine 214. In one exemplary embodiment (out of many), the total negative area of the six cut-outs includes approximately 1.5 mm-squared. The angle α between two adjacent spines 214 may be approximately 30 degrees to 100 degrees (e.g., approximately 60 degrees).

In FIG. 6B, this design has a small aperture 212A disposed at the center (coincident with longitudinal axis 86) of the basket 38 with a tadpole shaped cut-out 224 disposed on each of the spines 214. Each tadpole cut-out 224 is defined by an aperture cutout 212B that is merged with grooved cutout 212C. It is noted that while aperture 212A or 212B is shown approximating a circle, it is within the scope of this invention to have cut-out opening 212A or 212B in any shape as long as each aperture 212A or 212B has the requisite negative area. In the event the aperture 212A is configured as a circle, aperture 212A has central void A0 (of negative area) that can be approximated by a first virtual circle with radius r0 while each aperture 212B has a second area A2 that can be represented by a second virtual circle with radius r2. The apertures 212B (or the "heads" of the tadpole cutouts) are radially arrayed so that apertures 212B are contiguous to a first virtual circle with radius r1. The second virtual circle may have a second radius r2 of 1.2 times that of the radius r0 of the first virtual circle representing aperture 212A while the first virtual circle r1 may have radius r1 of approximately 1.5 times that of the radius of the central virtual circle r0. The tail or grooved opening 212C of the "tails" extends towards the proximal end of the basket 38 for a length L1 so that each tail is contiguous to an inside circumference of a third virtual circle 215. Slot length L1 includes approximately 6-10 times that of the first radius r1. Third virtual circle 215 may have a radius r3 extending from the longitudinal axis 86 where radius r3 includes approximately 10-15 times that of either first radius r1 or central radius r0. In the exemplary embodiment (amongst many), the negative area of each of the tadpole cutout 224 includes approximately 0.2 mm-squared while the negative area of center aperture 212A includes approximately 0.05mm-squared so that the total negative area defined by all of the cut-outs includes approximately 1.4mm-squared. In the same exemplary embodiment, the central radius r0 may be approximately 0.13mm, the second radius r2 may be approximately 0.2mm, and the first radius r1 may be approximately 0.23mm.

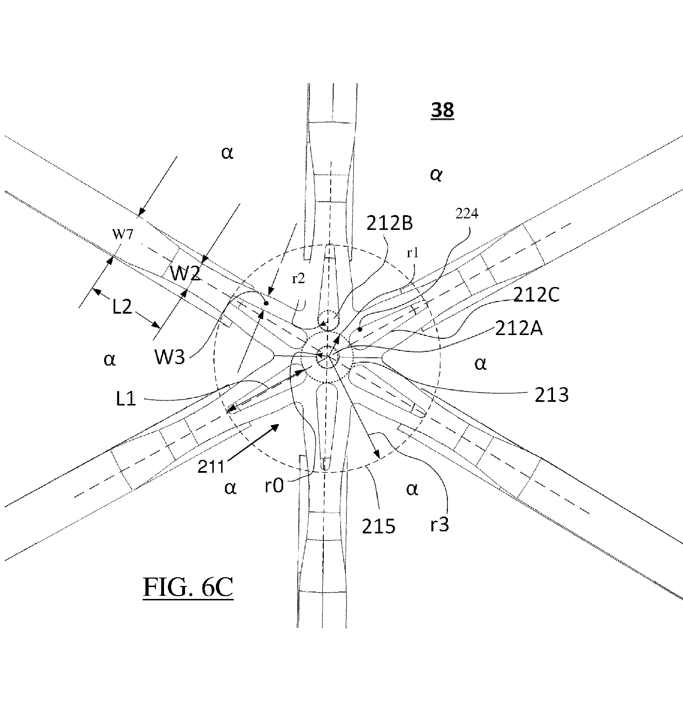

In FIG. 6C, the design of the basket 38 is provided with an aperture 212A at approximate center (i.e., axis 86) of the spines 214. Each spine 214 is provided a comet-shaped cutout 224 with head portion 212B with an open tapered slot tail 212C tapering towards the proximal portion of each spine 214. The comet-shaped cut-outs 212B are arrayed so that the distal head portion 212B of the cutout 224 are contiguous to an outside circumference of second virtual circle 213 while the proximal slotted opening 212C of the cutouts 224 are contiguous on the inside circumference of third virtual circle 215. Where the aperture 212A is configured as a circular hole located on central axis 86 with radius r0 where the second radius r2 includes approximately 90% of the central radius r0, the second virtual circle 213 may have a first radius r1 of approximately 2.5 times that of central radius r0 while the third virtual circle 215 has a radius r3 of approximately 10 times that of the central radius r0 (all measured from center axis 86). Spine 214 has a width W7 that tapers towards central axis 86 to a narrower second spine width W2 of approximately 66% of width W7 at its narrowest point before being sub-divided by comet shaped cut-out 212B into two narrower spine arms with each arm including a third spine width W3 of approximately 1/3 that of the width W7. The comet shaped cut-out 212B has a length L1 along the spine of approximately 1.8 times that of the largest spine width W7.

In FIG. 6D, the basket 38 has its distal portion configured to have an open center 212A that radiates into each of the six spines 214, each approximately 60 degrees apart. The open center 212A has a first area A1 that can be approximated by a virtual circle with radius r1. Three spines approximately 120 degrees apart have tapering grooves 212B extending back toward the proximal portion of basket 38. Three other spines approximately 120 degrees apart have large apertures 217 with area A3 disposed towards the proximal portion of the basket 38. The cut-out area A3 can be approximated by a virtual circle with radius r3 and disposed on the spines 214 such that the apertures 217 are contiguous to the inside circumference of virtual circle 215 with radius r2. In this configuration, each third area A3 is about 1/4 of the open first area A1 while the total negative surface area of the entire cut out includes approximately 1.6 times the first open area of empty space A1 and the second area A2 (calculated with radius r2) includes approximately 7 times the first area A1. Additionally, the second area A2 includes approximately 36 times third area A3. The radius r3 includes approximately 0.4 times that of radius r1 while radius r2 includes approximately 2.8 times that of radius r1. In one exemplary embodiment, first open area of empty space A1 includes approximately 2 mm-squared; second area A2 (as defined by radius r2) being approximately 15mm-squared; third area A3 includes approximately 0.4 mm-squared; total area of all cut-outs includes approximately 3.5 mm-squared; radius r1 ~ 0.8mm; r2 ~2.2mm; and r3 ~0.4mm.

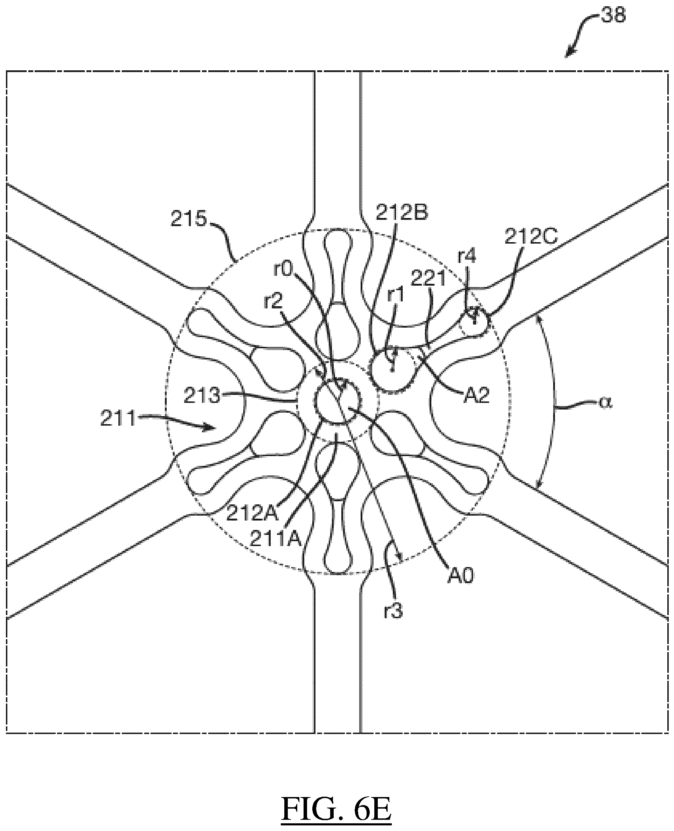

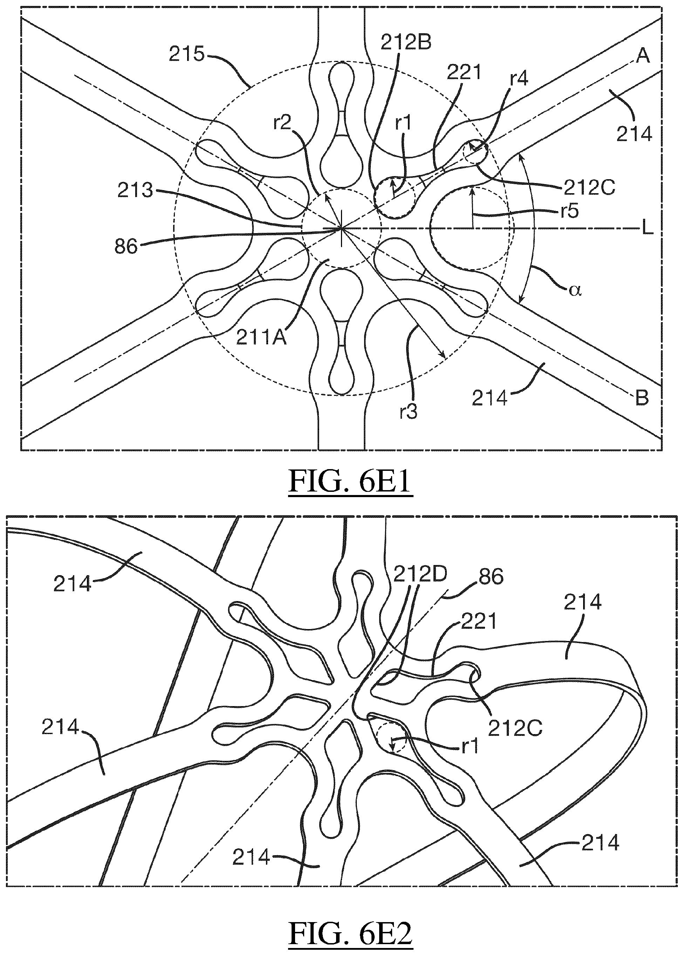

In FIG. 6E, the basket 38 may include a distal portion with an aperture 212A at approximate center (i.e., axis 86) of the spines 214 where the aperture 212A includes a radius r0 that may be about 0.13 mm to about 0.4 mm and a negative area A0 of approximately 0.01 mm-squared to approximately 0.4 mm-squared (e.g., 0.2 mm-squared). The spines 214 may be approximately 60 degrees apart at angle α and configured such that a fifth virtual circle of radius r5 is disposed between adjacent spines and the central distal portion 211A. The radius r5 of fifth virtual circle is substantially the same as the radius r1 of first virtual circle 213. The center of fifth virtual circle with radius r5 is located on an axis L bisecting the respective axes (e.g., A and B) of adjacent spines 214 (Fig. 6E1) such that any two adjacent spines 214 will have substantially the same fifth virtual circle with radius r5 disposed between them. The basket 38 may also include a plurality of hourglass cutouts 221 that extend radially along each spine 214. The hourglass cutouts 221 may include a major portion 212B at least primarily located at the central spine intersection 211 and may have a radius r1 approximately equal to the radius r0 of aperture 212A. The hourglass cutouts 221 may also include minor portion 212C located at the spines 214 which may have a radius r4 approximately half of the radius r1 of the major portion 212B. Each hourglass cutout 221 may include an area A2 of approximately 0.1 mm-squared to approximately 0.55 mm-squared (e.g., 0.39 mm squared) so that the total area of all the hourglass cutouts 221 may be 0.6 mm-squared to 3.3 mm-squared (e.g., 2.54 mm-squared). The major portions 212B are arrayed so that the major portions 212B are contiguous to an outside circumference of first virtual circle 213 with radius r1. The minor portions 212C are bounded by a third virtual circle 215 with a radius r3 approximately 10 times that of the first radius r1.

FIG. 6E1 is a variation of the embodiment shown in FIG. 6E and FIG. 6E1 uses the same nomenclatures as in Fig. 6E. In Fig. 6E1, the basket assembly does not utilize a central opening (i.e., a hole) 212A. All other features are the same as indicated by the same reference alphanumeric indicators.

FIG. 6E2 is a variation of the embodiment of FIG. 6E1 in which the cutout 212B is no longer circular but more of a snake-head like configuration referenced here as 212D. While the first virtual circle r1 can be seen disposed inside the cutout 212D, the cutout 212D elongates towards the central axis 86 such that cutout 212D would be insider the virtual circle r2 of FIG. 6E1. Cutout 212D tapers to a narrow portion and extends towards the proximal end of the assembly 38 to have the same cutout 212C disposed around the fourth virtual circle r4 with its open area as in the embodiment of FIG. 6E and FIG. 6E1.

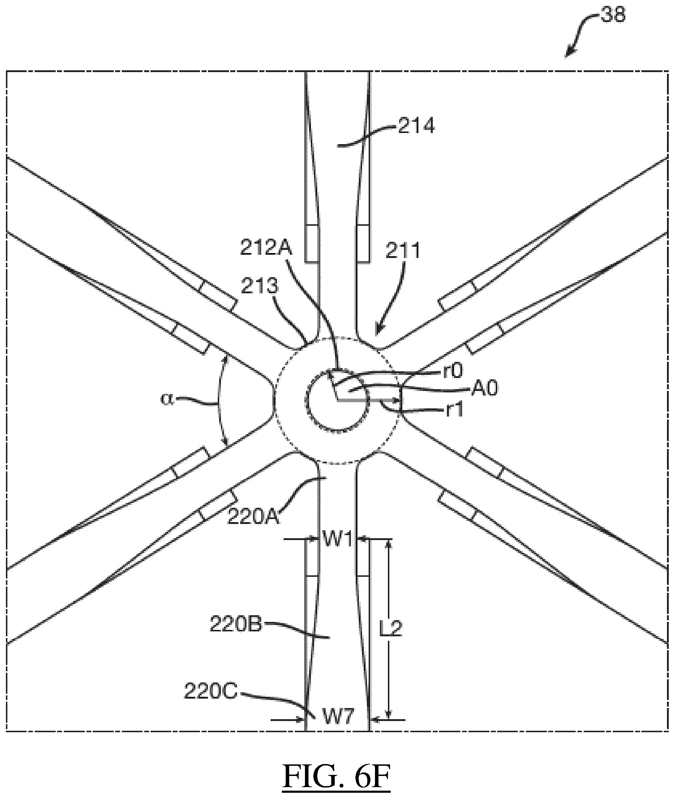

In FIG. 6F, the basket 38 may include a distal portion with an aperture 212A at the approximate center (i.e., axis 86) of the spines 214 or the central spine intersection 211 without any radial cutouts along the spines 214. The aperture 212a may include a first radius r0 approximately 0.1 mm to approximately 0.4mm and an area A0 of approximately 0.01 mm-squared to approximately 0.4 mm-squared (e.g., preferably 0.2 mm-squared). Each spine 214 may include a first portion 220A, a second portion 220B, and a third portion 220C. The first portion 220A may include a first width W1 of approximately 0.05 mm to approximately 0.65 mm (e.g., approximately 0.26 mm). The third portion 220C may include a width W7 of approximately 0.1 mm to approximately 1.0 mm (e.g., approximately 0.56 mm) and the second portion 220B may include a tapering width narrowing from the third portion 220C to the first portion 220A with an average width of approximately 0.2 mm to approximately 0.3 mm (e.g., approximately 0.31 mm). In some embodiments, the emergence of individual spines 214 may be defined by virtual circle 213 which may have a radius r1 of approximately double that of radius r0 of the aperture 212A.

In FIG. 6G, the basket 38 may include a distal end with an aperture 212A at the approximate center (i.e., axis 86) of the spines 214 or the central spine intersection 211. Much like other embodiments, the angle α between spines 214 may be approximately 60 degrees. The aperture 212A has a radius r1 of approximately 0.4 mm to approximately 1.2 mm (e.g., approximately 0.84 mm). The basket 38 may also include a plurality of radial cutouts 212B that extend from and are connected to the aperture 212A to form a single cutout, which may result from cutting the basket 38 from tubular stock rather than planer material. Regardless, each radial cutout 212B may include an ellipse shape at an end furthest from the aperture 212A. Each ellipse-shaped radial cutout 212B may include a length L4 of approximately 0.20 mm to approximately 0.55 mm and a width W5 of approximately 0.1 mm to approximately 0.45 mm. The ellipse-shaped radial cutouts 212B may be contiguous to the inside circumference of virtual circle 215 with radius r2. In this configuration, radius r2 may be approximately 1.5 times to approximately 3 times (e.g., approximately 2.4 times) the radius r1 of the aperture. Each spine 214 may include a thickness of approximately 0.03 mm to 0.15 mm (e.g., approximately 0.09 mm). Each spine 214 may also include two connecting portions 218 that connect to adjacent spines 214 and the width W5 of each connecting portion may be approximately 0.12 mm to approximately 0.4 mm (e.g., approximately 0.24 mm). Connecting portions 218 from adjacent spines 214 may be connected and may together form a circular shape that may have a radius r6 of approximately 0.25 mm to approximately 0.75 mm (e.g., approximately 52 mm).

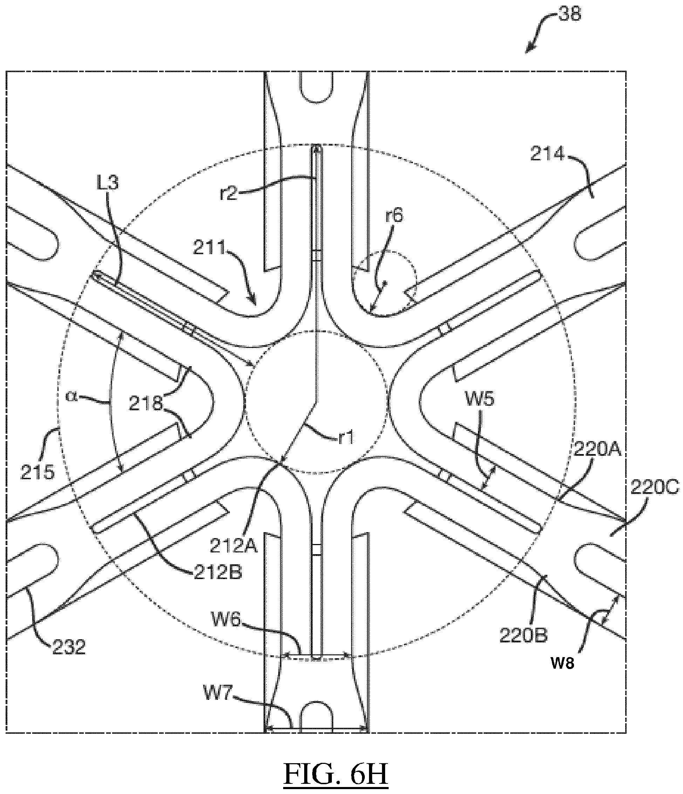

In FIG. 6H, the basket 38 may include a distal end with an aperture 212A at the approximate center (i.e., axis 86) of the spines 214 or the central spine intersection 211. Much like FIG. 6G, the angle α between spines 214 may be approximately 60 degrees, the aperture 212A may include a radius r1 of approximately 0.4 mm to approximately 1.2 mm (e.g., approximately 0.84 mm), and the basket 38 may include a plurality of radial cutouts 212B that extend from and are connected to the aperture 212A to form a single cutout, which may result from cutting the basket 38 from tubular stock rather than planer material. However, unlike FIG. 6G, the radial cutouts 212B may not include an ellipse shape and instead may be linear shaped with a length L3 of approximately 1 mm to approximately 2 mm, preferably about 1.5 mm and a generally even width but the linear struts may be tapered to distribute stresses and strains. The radial cutouts 212B may form connecting portions 218 in each spine 214 where each connecting portion 218 may have width W5 of approximately 0.12 mm to approximately 0.4 mm (e.g., approximately 0.24 mm) where the two adjacent connecting portions 218 are connected to form a circular shape between spines 214 that has a radius r6 of approximately 0.1 mm to approximately 1.1 mm (e.g., approximately 0.57 mm). The radial cutouts 212B may be contiguous to the inside circumference of virtual circle 215 with radius r2. In this configuration, radius r2 may be approximately 3.6 times the radius r1 of the aperture 212A. Each spine 214 may include the connecting portions 218, a first portion 220A with a width W6 of approximately 0.2 mm to approximately 0.9 mm (e.g., approximately 0.56 mm), a second portion 220B, and a third portion 220C with a width W7 of approximately 0.4 mm to approximately 1.2 mm (e.g., approximately 0.81 mm). The second portion 220B may have a tapering width tapers its width from width W7 to W6. Additionally, the third portion 220C of each spine may be a split spine that is split into two minor portions by slot 232, each minor portion having a width W8 of approximately 0.1 mm to approximately 0.6 mm (e.g., approximately 0.28 mm).

In FIG. 6I, the basket 38 may include a distal end with an aperture 212A at the approximate center (i.e., axis 86) of the spines 214 or at the central spine intersection 211. Much like FIG. 6G, the angle α between spines 214 may be approximately 60 degrees, the aperture 212A may include a radius r1 of approximately 0.2 mm to approximately 1.25 mm (e.g., approximately 0.745 mm), and the basket 38 may include a plurality of radial cutouts 212B that extend from and are connected to the aperture 212A to form a single cutout, which may result from cutting the basket 38 from tubular stock rather than planer material. The radial cutouts 212B may include a circular shape having a radius r4 of approximately 0.05 mm to approximately 0.6 mm (e.g., approximately 0.275 mm). The center of radius r4 is located at a distance L4 of approximately 1 mm to approximately 3mm from the longitudinal axis L-L and preferably approximately 2 mm. The radial cutouts 212B may form connecting portions 218 in each spine 214 where each connecting portion 218 may have width W5 of approximately 0.12 mm to approximately 0.4 mm (e.g., approximately 0.24 mm) and where the two adjacent connecting portions 218 are connected to form a circular shape between spines 214 that has a radius r6 of approximately 0.3 mm to approximately 1.7 mm (e.g., approximately 0.78 mm). The center of radius r6 is located at a distance L6 of approximately 0.5mm to approximately 3 mm from the central longitudinal axis L-L and preferably approximately 1.4 mm. The radial cutouts 212B may be contiguous to the inside circumference of virtual circle 215 with radius r2. In this configuration, radius r2 may be approximately 2.7 times the radius r1 of the aperture 212A. Each spine 214 may include the connecting portions 218 and a split portion extending away from the connecting portions 218 and the distal end of the basket 38. The split portion may include a slot 232 outside of the circumference of the virtual circle 215 that splits the spine 214 into two minor portions, each minor portion having a width W8 of approximately 0.1 mm to approximately 0.6 mm (e.g., approximately 0.28 mm). The width W7 of the split portion of the spine 214 may be approximately 0.4 mm to approximately 1.2 mm (e.g., approximately 0.81 mm) and the width W9 of the slot itself is approximately 0.05 mm to approximately 0.55 mm (e.g., approximately 0.25 mm).

In FIG. 6J, the basket 38 may include a distal end with spines 214 forming a spiral pattern at the central spine intersection 211 and an aperture 212A at the approximate center (i.e., axis 86) of the spines 214 or the central spine intersection 211. Each spine 214 may also include a first portion 220A as a spiral with a width W4 of approximately 0.01 mm to approximately 0.5 mm (e.g., approximately 0.26 mm), a second portion 22B with a width that gradually increase from width W4 to a width W7 of third portion 220C. The width W7 of the third portion 220C of the spine 214 may be approximately 0.01 mm to approximately 1.1 mm (e.g., approximately 0.56 mm). The first portion 220A of the spines 214 may be spiral arcs that with pitch angles β of approximately 60 degrees to approximately 120 degrees (e.g., approximately 90 degrees). The pitch angle is the angle formed between a tangent of a spiral arc 236 of a first portion 220A of a spine 214 and a tangent of a virtual circle 238 centered about the spiral pattern at the point where the two lines intersect. The angle α between spines 214 may be approximately 60 degrees and the center aperture 212A may include a radius r0 of approximately 0.01 mm to approximately 0.3 mm (e.g., approximately 0.125 mm).

FIG. 6K illustrates a similar design as shown in FIG. 6J except that the pitch angle β is larger at approximately 90 degrees to approximately 150 degrees (e.g., approximately 120 degrees).

The spines 214 can be folded or otherwise bent such that each respective attachment end 216 of the spine 214 can be inserted into the distal end 85 of the tubular shaft 84 (as shown in FIG. 2B) and relief lands 96 of spine retention hub 90 (not shown). Although not shown in FIGS. 5A and 5B, it will be appreciated that electrodes 40 can be attached to spines 214 before the spines are inserted into the tubular shaft 84 to form the basket assembly 38. As stated previously, the spines 214 can include a flexible, resilient material (e.g., a shape-memory alloy such as nickel-titanium, also known as Nitinol) that can enable the basket assembly 38 to transition to its expanded form (as shown in FIG. 2A) when the basket assembly 38 is deployed from tubular shaft 84. As will become apparent throughout this disclosure, spines 214 can be electrically isolated from electrode 40 to prevent arcing from electrode 40 to the respective spine 214.

As will be appreciated by one skilled in the art with the benefit of this disclosure, basket assembly 38 shown in FIGS. 2A through 2C including spines 214 formed from a single sheet of planar material and converging at a central intersection is offered merely for illustrative purposes and the disclosed technology can be applicable to other configurations of basket assemblies 38. For example, the described configuration of the basket spine assemblies can be obtained via laser cutting a nitinol tube and heat treating the spines from the tubular stock into substantially the planar form shown herein. As well, the disclosed technology can be applicable to basket assemblies 38 formed from a single spine 214 or multiple spines 214 with each spine 214 being attached at both ends. In other examples, the basket assembly 38 can include a central hub connecting the multiple spines 214 together at a distal end 39 of the basket assembly 38. In yet other examples, the basket assembly 38 can include a single spine 214 configured to form a spiral, multiple spines 214 configured to form a spiral, multiple spines 214 configured to form a tripod or multiple tripods, or any other shape of basket assembly 38. Thus, although FIGS. 2A through 2C illustrate a specific configuration of basket assembly 38, the disclosed technology should not be construed as so limited.

In the exemplary embodiments shown herein, the spines width W may have a nominal width of approximately 0.6 mm and can be as low as 0.2 mm or as large as 1.5 mm. The thickness of each spine can be nominally 0.09 mm and can vary from 0.05mm to 0.2mm. It should be noted that these values for width and thickness can vary depending on the stiffness desired.

Referring back to FIG. 2A through FIG. 2C, one or more electrodes 40 can be attached to spines 214 to form the basket assembly 38. In some examples, each electrode 40 can include electrically conductive material (e.g., gold, platinum and palladium (and their respective alloys)).

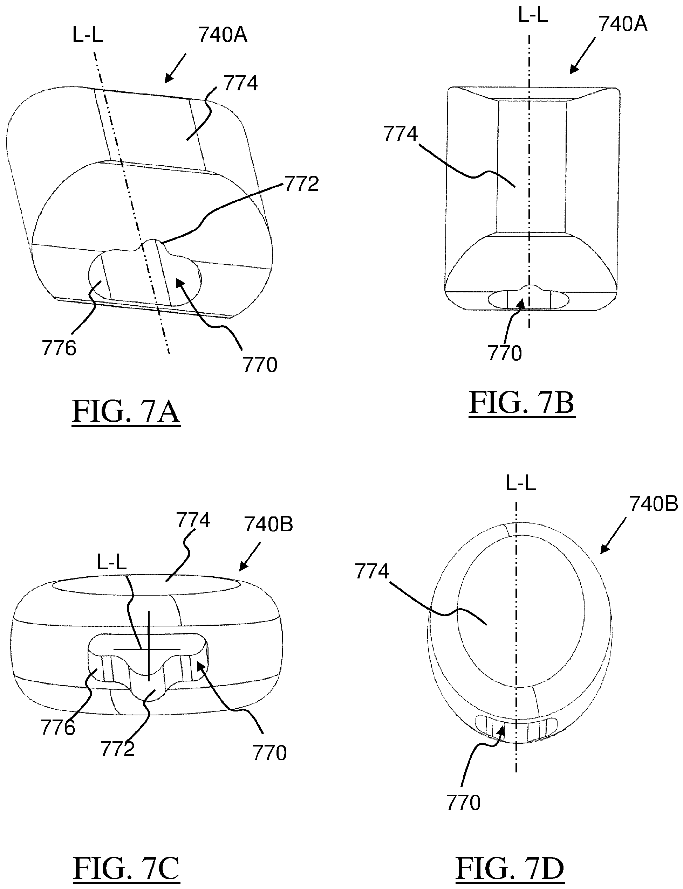

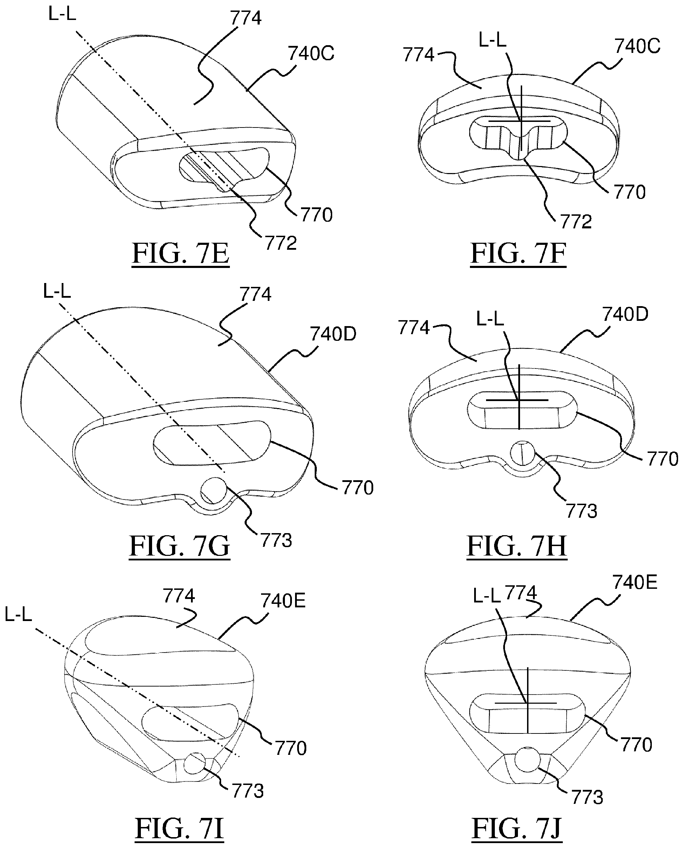

Turning to FIGS. 7A through 7J, electrode 40 can have a variety of cross-sectional shapes, curvatures, lengths, lumen number and lumen shape as provided as examples in electrodes 740A-740E. The electrodes 740A-740E are offered to illustrate various configurations of electrodes 40 that can be used with the medical device 22 but should not be construed as limiting. One skilled in the art will appreciate that various other configurations of electrodes 40 can be used with the disclosed technology without departing from the scope of this disclosure.

Each electrode 740A-740E can have an outer surface 774 facing outwardly from electrode 740 and an inner surface 776 facing inwardly toward electrode 740 where at least one lumen 770 is formed through electrode 740. The lumen 770 can be sized and configured to receive a spine 214 such that spine 214 can pass through electrode 740. Lumen 770 can be a symmetric opening through electrode 740A-740E and can be disposed offset with respect to a longitudinal axis L-L of the respective electrode. In other examples, lumen 770 can pass through electrode 740 in a generally transverse direction with respect to the longitudinal axis L-L of the respective electrode. Furthermore, lumen 770 can be positioned in electrode 740 nearer a bottom surface, nearer a top surface, or nearer a middle of electrode 740 depending on the particular configuration. In FIGS. 7A, 7C, and 7E through 7J, the top surface (upper side) is oriented toward the top of the drawing, the bottom surface (lower side) is oriented toward the bottom of the drawing, and the middle is between the top surface and the bottom surface. In other words, each electrode 740A-740E can include a lumen 770 that is offset with respect to a centroid of the electrode740A-740E.

In addition, as shown in FIGS. 7A through 7F, electrodes 740A-740C can have a wire relief 772 forming a recess or depression in electrode 740 adjacent lumen 770 for one or more wires to pass through lumen 770 along with a respective spine 214. Relief 772 can be sized to provide room for a wire of electrode 740 to pass through electrode 740 such that electrode 740 can be in electrical communication with the control console 24.

Alternatively, or in addition thereto, wires can pass through a wire lumen 773 as shown in example electrodes 740D and 740E in FIGS. 7G through 7J. Although not depicted, electrodes 40 may include both a wire relief 772 adjacent lumen 770 and wire lumen 773. Such electrode may permit additional wires to pass through the electrode body.

As shown in FIGS. 7A-7J, the electrodes 740A-740E can include various shapes depending on the application. For example, as illustrated in FIGS. 7A and 7B, the electrode 740A can have a substantially rectangular cuboid shape with rounded edges. In other examples, the electrode 740B can have a substantially ovoid shape (as illustrated in FIGS. 7C and 7D), the electrode 740C, 740D can have a contoured shape including a convex side and a concave side (as illustrated in FIGS. 7E through 7H), or the electrode 740E can have a contoured shape including substantially more material proximate an upper side than a lower side of the electrode 740E (as illustrated in FIGS. 7I and 7J). As will be appreciated by one of skill in the art, the various example electrodes 740A-740E shown in FIGS. 7A-7J, and described herein, are offered for illustrative purposes and should not be construed as limiting.

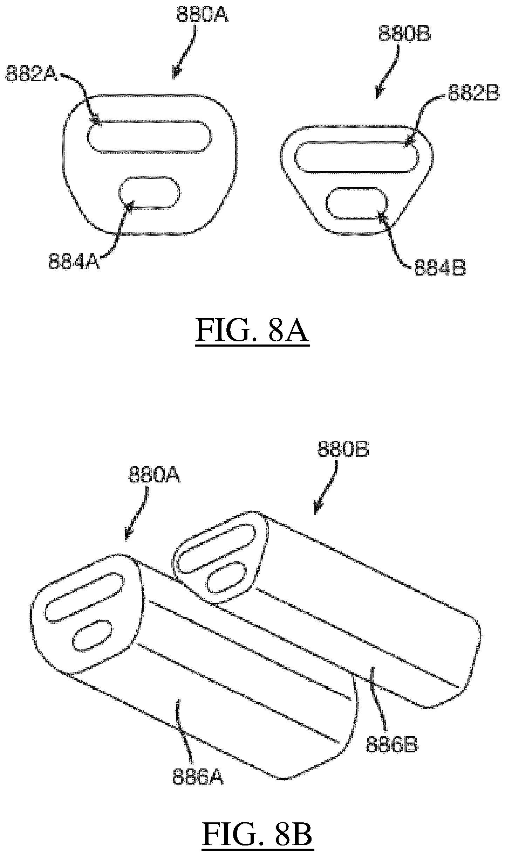

FIGS. 8A and 8B are schematic pictorial illustrations showing various insulative jackets 880A, 880B of a given medical device 22. FIG. 8A is a front view while FIG. 8B is a perspective view of insulative jackets 880A, 880B. Insulative jackets 880A, 880B can be made from a biocompatible, electrically insulative material such as polyamide-polyether (Pebax) copolymers, polyethylene terephthalate (PET), urethanes, polyimide, parylene, silicone. In some examples, insulative material can include biocompatible polymers including, without limitation, polyetheretherketone (PEEK), polyglycolic acid (PGA), poly (lactic-co-glycolic acid) copolymer (PLGA), polycaprolactive (PCL), poly(3-hydroxybutyrate-co-3-hydroxyvalerate) (PHBV), poly-L-lactide, polydioxanone, polycarbonates, and polyanhydrides with the ratio of certain polymers being selected to control the degree of inflammatory response. Insulative jackets 880A, 880B may also include one or more additives or fillers, such as, for example, polytetrafluoroethylene (PTFE), boron nitride, silicon nitride, silicon carbide, aluminum oxide, aluminum nitride, zinc oxide, and the like. Insulative jacket 880A, 880B can help to insulate a spine 214 and/or wires passing through insulative jacket 880A, 880B from electrode 40 to prevent arcing from electrode 40 to the spine 214 and/or mechanical abrasion of wires passing through insulative jacket 880A, 880B.

As illustrated in FIGS. 8A and 8B, insulative jackets 880A, 880B, can include a cross-sectional shape that is substantially trapezoidal. The insulative jacket may consist of a single lumen or multi-lumen configuration. Multi-lumen jackets may be configured such that the alloy frame and wires share a single lumen while the second lumen may be used for irrigation. The alloy frame and wires may occupy separate lumens, also, as described. The current embodiment does not utilize irrigated jackets. For these designs, the insulative jackets may be continuous (individual sleeves extending from proximal to distal end of each alloy frame strut), segmented (bridging between electrode gaps), or a combination of both. Furthermore, insulative jacket 880A, 880B can include a first lumen 882A, 882B and a second lumen 884A, 884B. First lumen 882A, 882B can be configured to receive spine 214 while second lumen 884A, 884B can be configured to receive a wire, or vice-versa. In other examples, first lumen 882A, 882B and second lumen 884A, 884B can each be configured to receive one or more wires that can be connected to one or more electrodes 40. Furthermore, as illustrated in FIG. 8B, insulative jacket 880A, 880B can include an aperture 886A, 886B through which a wire can be electrically connected to electrode 40. Although illustrated in FIG. 8B as being proximate a bottom of insulative jacket 880A, 880B, aperture 886A, 886B can be positioned proximate a top or side of insulative jacket 880A, 880B. Furthermore, insulative jacket 880A, 880B can include multiple apertures 886A, 886B with each aperture being disposed on the same side of insulative jacket (i.e., top, bottom, left, right) or on different sides of the insulative jacket depending on the application.

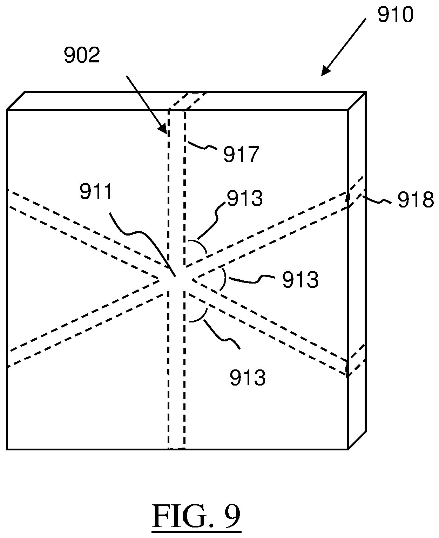

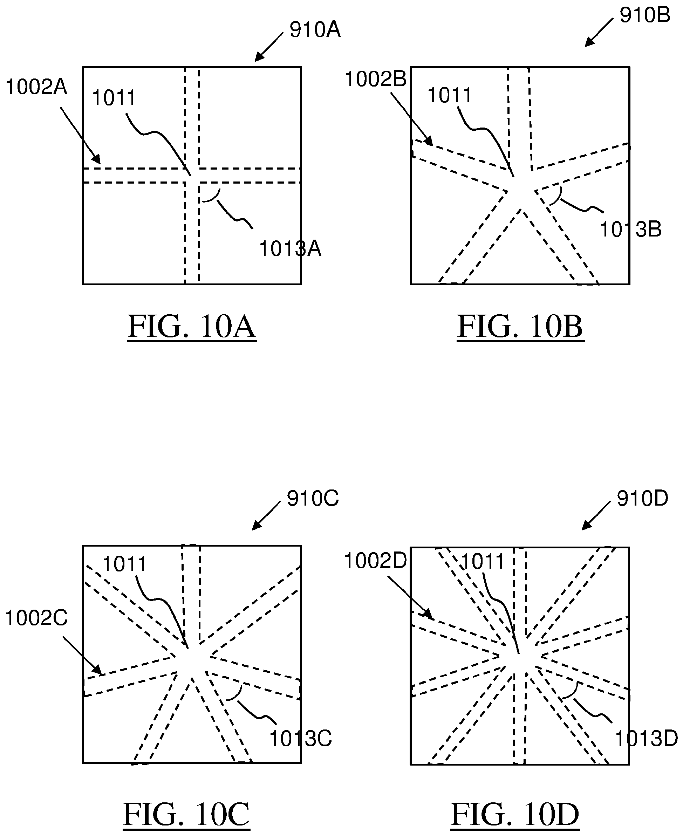

FIGS. 9 and 10A-10D are schematic pictorial illustrations of cutting patterns for various linear spines patterns 902 from a planar sheet of material 910. As described supra, planar sheet of material 910 can include a number of spines 214 ranging from about four to about ten spines. As illustrated in FIG. 9, planar sheet of material 910 can include central intersection 911 and spine pattern 902, which includes one or both of longitudinal scores 917 and transverse scores 918. In any of the embodiments disclosed herein, planar sheet of material 910 can also include a central intersection 911 and spine patterns 902 including an equiangular pattern 913. Planar sheet of material 910 can include spine patterns including a number of spine patterns 902 forming spines 214 in basket assembly 38. As would be understood by one of skill in the art, adjusting the number of spine patterns 902 may impact a number of factors including, without limitation, stability, flexibility, surface contact, and ablation capacity of medical probe 22.

FIGS. 10A through 10D provide example spine patterns 1002A, 1002B, 1002C, 1002D, although additional spine patterns are contemplated. Similar to the above planar sheet of material 910, spine patterns 1002A-1002D can include a respective central intersection 1011 and a respective equiangular pattern 1013A-1013D. As would be appreciated by one of skill in the art, as the number of spines added to spine pattern 1002A-1102D, the angle for equiangular pattern 1013A-1013D may change. In each of these examples provided, planar sheet of material 910A, 910B, 910C, 910D may also include central intersections and spine patterns including equiangular patterns. Although not depicted in FIGS. 10A-10D, planar sheet of material 910A-910D can include one or both of longitudinal scores 917 and transverse scores 918.

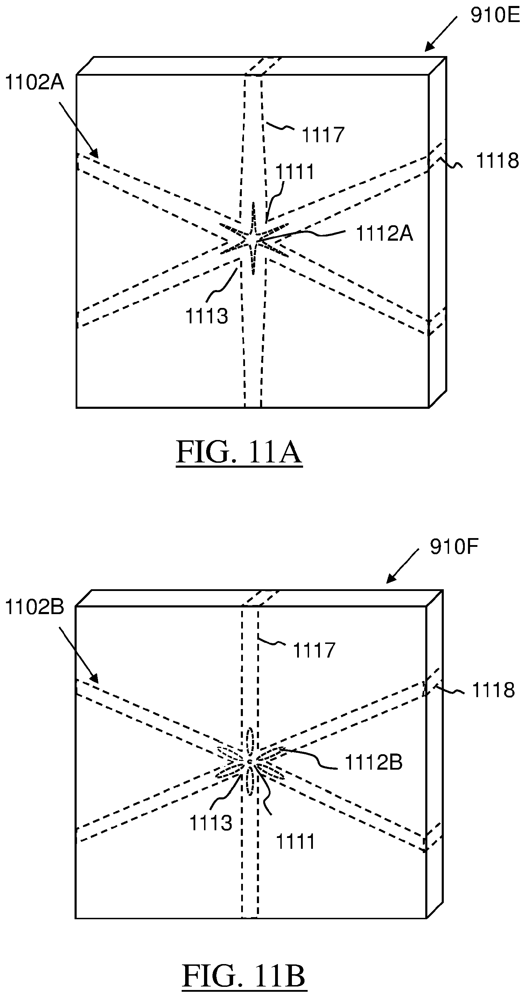

FIGS. 11A and 11B are schematic pictorial illustrations of cutting patterns for various linear spine patterns including one or more cutouts at a central spine intersection from a planar sheet of material. As described supra, planar sheet of material 910E, 910F may include a spine pattern 1102A including one cutout 1112A at central intersection 1111 or a spine pattern 1102B including two or more cutouts 1112B at central intersection 1111. As illustrated in FIGS. 11A and 11B, planar sheet of material 910E and 910F can include one or both of longitudinal scores 1117 and transverse scores 1118.

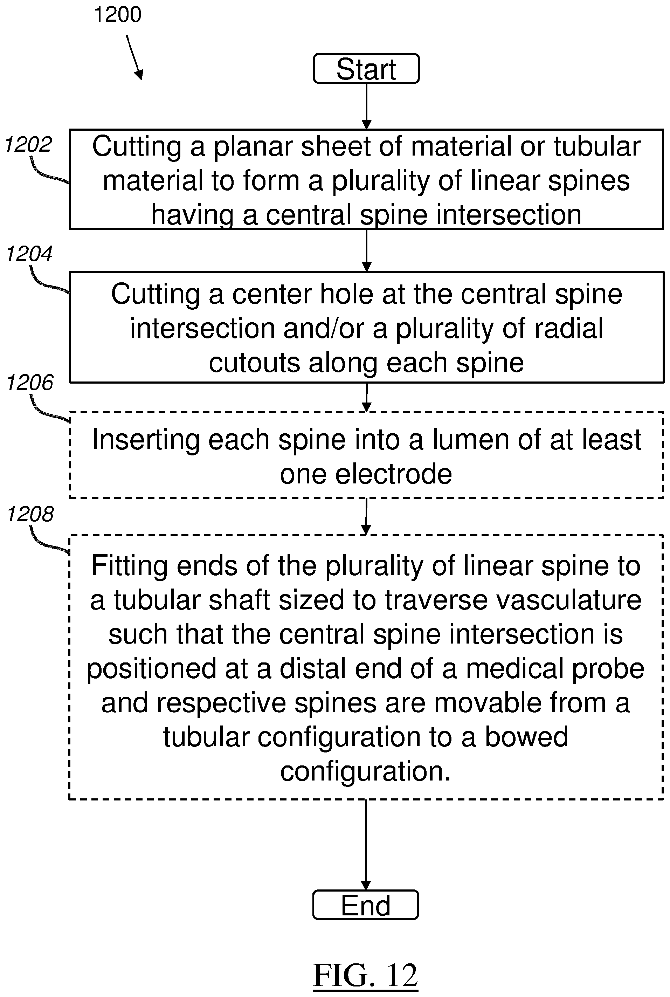

FIG. 12 is a flowchart illustrating a method 1200 of manufacturing a basket assembly 38, in accordance with an embodiment of the present invention. Method 1300 can include cutting 1202 a planar sheet of material 910 or a tubular material to form a plurality of linear spines 214 including a central spine intersection 211. Cutting 1202 the plurality of linear spines 214 can include cutting from a pattern 9002 (or 1002A-1002D) including longitudinal and transverse scores 917, 918. The planar sheet of resilient material can include shape-memory alloy such as nickel-titanium (also known as Nitinol), cobalt chromium, or any other suitable material. Method 1200 can include cutting 1204 one or more discrete cutouts 212 at the central spine intersection 211. As described supra, the one or more discrete cutouts 212 can be a single cutout or two or more cutouts. In addition, the one or more discrete cutouts 212can be cut in a pattern to extend along at least a portion of each spine. In some examples, steps 1202 and 1204 may occur as simultaneous steps or as a sequence of steps. As an alternative to steps 1202 and 1204, metallic strands can be shaped similar to the pattern formed by cutting the planar sheet in steps 1202 and 1204.

Method 1200 can optionally include inserting 1206 each spine into a lumen of at least one electrode. The electrodes can be positioned such that the electrodes are offset between electrodes on adjacent spines. Method 1200 can optionally include fitting 1308 ends of the plurality of linear spines to a tubular shaft sized to traverse vasculature such that the central spine intersection is positioned at a distal end of the medical probe and respective spines are movable from a tubular configuration to a bowed configuration. As will be appreciated by one of skill in the art including the benefit of this disclosure, fitting 1208 an end of the spine into a tubular shaft can include attaching the spine 214 to a spine retention hub 90. Furthermore, the spine retention hub 90 and/or the spine 214 and the tubular shaft 84 can be inserted into a flexible insertion tube 30 to form the medical probe 22

In some examples, the method can also include forming an approximately spheroid or oblate-spheroid shape with the linear spines. Method 1200 can further include electrically connecting the wire to the one or more electrodes. Method 1200 can also include disposing an insulative sleeve over the linear spines and within the lumen of the respective electrode.

As will be appreciated by one skilled in the art, method 1200 can include any of the various features of the disclosed technology described herein and can be varied depending on the particular configuration. Thus, method 1200 should not be construed as limited to the particular steps and order of steps explicitly described herein. It is noted that while the preference for the exemplary embodiments of the medical probe is for IRE or PFA, the medical probe may also be used separately only for RF ablation (unipolar mode with an external grounding electrode or bipolar mode) or in combination with IRE and RF ablations sequentially (certain electrodes in IRE mode and other electrodes in RF mode) or simultaneously (groups of electrodes in IRE mode and other electrodes in RF mode).

The embodiments described above are examples, and the present invention is not limited by what has been particularly shown and described hereinabove. Rather, the scope of the invention is defined by the appended claims.

## Claims

1. An expandable basket assembly (38) for a medical probe, comprising:
a single unitary structure comprising a plurality of spines (214) converging at a central spine intersection (211), the central spine intersection being positioned on a longitudinal axis of the expandable basket assembly at a distal end thereof; and
a plurality of radial cutouts (212) with each radial cutout defining an opening in each of the plurality of spines proximate the central spine intersection so that each opening extends for a length along each spine away from the central spine intersection, **characterised in that** each cut-out comprises a tadpole shaped cutout (224), the tadpole cutout includes a head portion (212B) contiguous to a circumference of a first virtual circle (213) with a first radius (r1) disposed about the longitudinal axis, the head portion defining a negative area approximating a second virtual circle with a second radius (r2), the head portion connected to a slotted tail portion (212C) extending for a first length (L1) along the spine and contiguous to an inside circumference of a third virtual circle (215) having a third radius (r3).

2. The expandable basket assembly of claim 1, wherein the first length (L1) of the slotted tail portion is approximately 6-10 times that of the length of the radius (r1) of the first virtual circle.

3. The expandable basket assembly of claim 2, further comprising a cutout disposed on the longitudinal axis to define a central negative area (A0) of a center aperture approximating a central circle including a central radius (r0) smaller than the first radius (r1).

4. The expandable basket assembly of claim 3, in which the negative area (A0) of each of the tadpole cutout comprises approximately 0.2 mm-squared while the negative area of center aperture (212A) is approximately 0.05mm-squared so that a total negative area defined by all of the cut-outs is approximately 1.4mm-squared.

5. The expandable basket assembly of claim 3, wherein central void radius (r0) comprises approximately 0.13mm, the second radius (r2) comprises approximately 0.2mm, and the first radius (r1) comprises approximately 0.23mm.

6. An expandable basket assembly (38) for a medical probe, comprising:
a single unitary structure comprising a plurality of spines (214) converging at a central spine intersection (211), the central spine intersection being positioned on a longitudinal axis of the expandable basket assembly at a distal end thereof;
a plurality of radial cutouts (212) with each radial cutout defining an opening in each of the plurality of spines proximate the central spine intersection so that each opening extends for a length along each spine away from the central spine intersection; and
**characterised by** further comprising a center aperture (212A) disposed on the central spine intersection, wherein the plurality of radial cutouts are separated from the center aperture by a portion of the central spine intersection.

7. The expandable basket assembly according to claim 6, wherein the center aperture comprises an area of about 0.01 mm-squared to about 0.4 mm-squared, optionally wherein each of the plurality of radial cutouts includes an area of about 0.1 mm-squared to about 0.55 mm-squared and each of the spines includes a first portion proximate to the central spine intersection with a first width, a second portion proximate the first portion with a second width less than the first portion, and a third portion proximate the second portion with a third width that is greater than the first width and greater than the second width, further optionally in which the cutout defines a comet-shaped cutout (224) with head portion (212B) with a slotted tapered tail (212C) extending to the proximal portion of each spine, still further optionally wherein the first width is about 0.15 mm to about 0.5 mm, the second width is about 0.05 mm to about 0.35 mm, and the third width is about 0.3 mm to about 0.7 mm, and the third portion of each spine comprises an electrode attachment slot configured to accept an electrode, the electrode attachment slot bisects each spine into two minor widths of about 0.05 mm to about 6 mm.

8. An expandable basket assembly (38) for a medical probe, comprising:
a single unitary structure comprising a plurality of spines (214) converging at a central spine intersection (211), the central spine intersection being positioned on a longitudinal axis of the expandable basket assembly at a distal end thereof; and
a plurality of radial cutouts (212) with each radial cutout defining an opening in each of the plurality of spines proximate the central spine intersection so that each opening extends for a length along each spine away from the central spine intersection, **characterised in that** each cut-out comprises two teardrop cutouts attached at their narrow portions to define a single cutout on each spine.

9. The expandable basket assembly according to claim 8, each cutout including an area of about 0.4 mm-squared.

10. An expandable basket assembly (38) for a medical probe, comprising:
a single unitary structure comprising a plurality of spines (214) converging at a central spine intersection (211), the central spine intersection being positioned on a longitudinal axis of the expandable basket assembly at a distal end thereof; and
a plurality of radial cutouts (212) with each radial cutout defining an opening in each of the plurality of spines proximate the central spine intersection so that each opening extends for a length along each spine away from the central spine intersection,
wherein each spine comprises two connecting portions (218), wherein the radial cutout forms the two connecting portions (218) in each spine,
**characterised in that** each spine further comprises a split portion extending away from the connecting portions and the distal end of the basket, wherein i) the split portion of each spine comprises a slot (232) that bisects each spine into two minor widths (W8) each of which comprises a width of approximately 0.1 mm to approximately 6 mm, or ii) the split portion of each spine comprises a slot (232) that bisects each spine into two minor portions with the slot comprising a width (W9) of approximately 0.05 mm to approximately 0.55 mm and the center aperture includes a radius of approximately 0.4 mm to approximately 1.2 mm, optionally wherein each radial cutout comprises an ellipse shape at an end furthest from the center aperture and the ellipse shape includes a length of about 0.20 mm to about 0.55 mm and a width of about 0.1 mm to about 0.45 mm.

11. An expandable basket assembly for a medical probe, comprising:
a single unitary structure that includes a plurality of spines converging at a central spine intersection, the central spine intersection being positioned on a longitudinal axis of the expandable basket assembly at a distal end thereof, **characterised by** the plurality of spines converging at the central spine intersection in a spiral pattern, wherein each of the plurality of spines includes a first width (W7) extending away from the distal end and a second width (W4) associated with the spiral pattern and narrower than the first width.

12. The expandable basket assembly of claim 11, wherein the first width is approximately 0.1 mm to approximately 1.0 mm, the second width is approximately 0.05 mm to approximately 0.65 mm, and each spine comprises a tapering width disposed between the first and second widths.

13. A method of manufacturing the basket assembly of any of claims 1-10, the method comprising:
cutting a planar sheet of material (910) to form a plurality of linear spines (214) having a central spine intersection (211);
cutting the plurality of radial cutouts; and
forming an approximately spheroid or oblate-spheroid shape with the linear spines.

## Patentansprüche

1. Expandierbare Korbanordnung (38) für eine medizinische Sonde, umfassend:
eine einzige einstückige Struktur, umfassend eine Vielzahl von Streben (214), die an einem zentralen Strebenkreuzungspunkt (211) zusammenlaufen, wobei der zentrale Strebenkreuzungspunkt auf einer Längsachse der expandierbaren Korbanordnung an einem distalen Ende davon positioniert ist; und
eine Vielzahl von radialen Ausschnitten (212), wobei jeder radiale Ausschnitt eine Öffnung in jeder der Vielzahl von Streben nahe dem zentralen Strebenkreuzungspunkt definiert, so dass sich jede Öffnung über eine Länge entlang jeder Strebe von dem zentralen Strebenkreuzungspunkt weg erstreckt, **dadurch gekennzeichnet, dass** jeder Ausschnitt einen kaulquappenförmigen Ausschnitt (224) umfasst, wobei der Kaulquappenausschnitt einen Kopfabschnitt (212B) einschließt, der an einen Umfang eines ersten virtuellen Kreises (213) mit einem ersten Radius (r1), der um die Längsachse herum angeordnet ist, angrenzt, wobei der Kopfabschnitt eine Negativfläche definiert, die ungefähr einem zweiten virtuellen Kreis mit einem zweiten Radius (r2) entspricht, wobei der Kopfabschnitt mit einem geschlitzten Schwanzabschnitt (212C) verbunden ist, der sich über eine erste Länge (L1) entlang der Strebe erstreckt und an einen Innenumfang eines dritten virtuellen Kreises (215) mit einem dritten Radius (r3) angrenzt.

2. Expandierbare Korbanordnung nach Anspruch 1, wobei die erste Länge (L1) des geschlitzten Schwanzabschnitts ungefähr das 6- bis 10-Fache der Länge des Radius (r1) des ersten virtuellen Kreises beträgt.

3. Expandierbare Korbanordnung nach Anspruch 2, ferner umfassend einen Ausschnitt, der auf der Längsachse angeordnet ist, um eine zentrale Negativfläche (A0) einer zentralen Apertur zu definieren, die ungefähr einem zentralen Kreis entspricht, der einen zentralen Radius (r0) einschließt, der kleiner als der erste Radius (r1) ist.

4. Expandierbare Korbanordnung nach Anspruch 3, wobei die Negativfläche (A0) jedes Kaulquappenausschnitts ungefähr 0,2 mm² umfasst, während die Negativfläche der zentralen Apertur (212A) ungefähr 0,05 mm² beträgt, so dass eine Gesamtnegativfläche, die durch alle Ausschnitte definiert ist, ungefähr 1,4 mm² beträgt.

5. Expandierbare Korbanordnung nach Anspruch 3, wobei ein zentraler Leerraumradius (r0) ungefähr 0,13 mm umfasst, der zweite Radius (r2) ungefähr 0,2 mm umfasst und der erste Radius (r1) ungefähr 0,23 mm umfasst.

6. Expandierbare Korbanordnung (38) für eine medizinische Sonde, umfassend:
eine einzige einstückige Struktur, umfassend eine Vielzahl von Streben (214), die an einem zentralen Strebenkreuzungspunkt (211) zusammenlaufen, wobei der zentrale Strebenkreuzungspunkt auf einer Längsachse der expandierbaren Korbanordnung an einem distalen Ende davon positioniert ist;
eine Vielzahl von radialen Ausschnitten (212), wobei jeder radiale Ausschnitt eine Öffnung in jeder der Vielzahl von Streben nahe dem zentralen Strebenkreuzungspunkt definiert, so dass sich jede Öffnung über eine Länge entlang jeder Strebe von dem zentralen Strebenkreuzungspunkt weg erstreckt; und
**gekennzeichnet durch** ferner umfassend eine zentrale Apertur (212A), die auf dem zentralen Strebenkreuzungspunkt angeordnet ist, wobei die Vielzahl von radialen Ausschnitten durch einen Abschnitt des zentralen Strebenkreuzungspunkts von der zentralen Apertur getrennt ist.

7. Expandierbare Korbanordnung nach Anspruch 6, wobei die zentrale Apertur eine Fläche von etwa 0,01 mm² bis etwa 0,4 mm² umfasst, optional wobei jeder der Vielzahl von radialen Ausschnitten eine Fläche von etwa 0,1 mm² bis etwa 0,55 mm² einschließt und jede der Streben einen ersten Abschnitt nahe dem zentralen Strebenkreuzungspunkt mit einer ersten Breite, einen zweiten Abschnitt nahe dem ersten Abschnitt mit einer zweiten Breite, die kleiner als der erste Abschnitt ist, und einen dritten Abschnitt nahe dem zweiten Abschnitt mit einer dritten Breite einschließt, die größer als die erste Breite und größer als die zweite Breite ist, ferner optional wobei der Ausschnitt einen kometenförmigen Ausschnitt (224) mit einem Kopfabschnitt (212B) mit einem geschlitzten, sich verjüngenden Schwanz (212C) definiert, der sich zu dem proximalen Abschnitt jeder Strebe erstreckt, noch ferner optional wobei die erste Breite etwa 0,15 mm bis etwa 0,5 mm beträgt, die zweite Breite etwa 0,05 mm bis etwa 0,35 mm beträgt und die dritte Breite etwa 0,3 mm bis etwa 0,7 mm beträgt und der dritte Abschnitt jeder Strebe einen Elektrodenbefestigungsschlitz umfasst, der dazu ausgelegt ist, eine Elektrode aufzunehmen, wobei der Elektrodenbefestigungsschlitz jede Strebe in zwei kleinere Breiten von etwa 0,05 mm bis etwa 6 mm halbiert.

8. Expandierbare Korbanordnung (38) für eine medizinische Sonde, umfassend:
eine einzige einstückige Struktur, umfassend eine Vielzahl von Streben (214), die an einem zentralen Strebenkreuzungspunkt (211) zusammenlaufen, wobei der zentrale Strebenkreuzungspunkt auf einer Längsachse der expandierbaren Korbanordnung an einem distalen Ende davon positioniert ist; und
eine Vielzahl von radialen Ausschnitten (212), wobei jeder radiale Ausschnitt eine Öffnung in jeder der Vielzahl von Streben nahe dem zentralen Strebenkreuzungspunkt definiert, so dass sich jede Öffnung über eine Länge entlang jeder Strebe von dem zentralen Strebenkreuzungspunkt weg erstreckt, **dadurch gekennzeichnet, dass** jeder Ausschnitt zwei tropfenförmige Ausschnitte umfasst, die an ihren schmalen Abschnitten aneinander befestigt sind, um einen einzigen Ausschnitt an jeder Strebe zu definieren.

9. Expandierbare Korbanordnung nach Anspruch 8, wobei jeder Ausschnitt eine Fläche von etwa 0,4 mm² einschließt.

10. Expandierbare Korbanordnung (38) für eine medizinische Sonde, umfassend:
eine einzige einstückige Struktur, umfassend eine Vielzahl von Streben (214), die an einem zentralen Strebenkreuzungspunkt (211) zusammenlaufen, wobei der zentrale Strebenkreuzungspunkt auf einer Längsachse der expandierbaren Korbanordnung an einem distalen Ende davon positioniert ist; und
eine Vielzahl von radialen Ausschnitten (212), wobei jeder radiale Ausschnitt eine Öffnung in jeder der Vielzahl von Streben nahe dem zentralen Strebenkreuzungspunkt definiert, so dass sich jede Öffnung über eine Länge entlang jeder Strebe von dem zentralen Strebenkreuzungspunkt weg erstreckt,
wobei jede Strebe zwei Verbindungsabschnitte (218) umfasst, wobei der radiale Ausschnitt die zwei Verbindungsabschnitte (218) in jeder Strebe bildet,
**dadurch gekennzeichnet, dass** jede Strebe ferner einen geteilten Abschnitt umfasst, der sich von den Verbindungsabschnitten und dem distalen Ende des Korbs weg erstreckt, wobei i) der geteilte Abschnitt jeder Strebe einen Schlitz (232) umfasst, der jede Strebe in zwei kleinere Breiten (W8) halbiert, von denen jede eine Breite von ungefähr 0,1 mm bis ungefähr 6 mm umfasst, oder ii) der geteilte Abschnitt jeder Strebe einen Schlitz (232) umfasst, der jede Strebe in zwei kleinere Abschnitte halbiert, wobei der Schlitz eine Breite (W9) von ungefähr 0,05 mm bis ungefähr 0,55 mm umfasst und die zentrale Apertur einen Radius von ungefähr 0,4 mm bis ungefähr 1,2 mm einschließt, optional wobei jeder radiale Ausschnitt an einem Ende, das am weitesten von der zentralen Apertur entfernt ist, eine Ellipsenform umfasst und die Ellipsenform eine Länge von etwa 0,20 mm bis etwa 0,55 mm und eine Breite von etwa 0,1 mm bis etwa 0,45 mm einschließt.

11. Expandierbare Korbanordnung für eine medizinische Sonde, umfassend:
eine einzige einstückige Struktur, die eine Vielzahl von Streben einschließt, die an einem zentralen Strebenkreuzungspunkt zusammenlaufen, wobei der zentrale Strebenkreuzungspunkt auf einer Längsachse der expandierbaren Korbanordnung an einem distalen Ende davon positioniert ist, **gekennzeichnet durch** das Zusammenlaufen der Vielzahl von Streben an dem zentralen Strebenkreuzungspunkt in einem Spiralmuster, wobei jede der Vielzahl von Streben eine erste Breite (W7), die sich von dem distalen Ende weg erstreckt, und eine zweite Breite (W4) einschließt, die dem Spiralmuster zugeordnet und schmaler als die erste Breite ist.

12. Expandierbare Korbanordnung nach Anspruch 11, wobei die erste Breite ungefähr 0,1 mm bis ungefähr 1,0 mm beträgt, die zweite Breite ungefähr 0,05 mm bis ungefähr 0,65 mm beträgt und jede Strebe eine sich verjüngende Breite umfasst, die zwischen der ersten und der zweiten Breite angeordnet ist.

13. Verfahren zum Herstellen der Korbanordnung nach einem der Ansprüche 1 bis 10, das Verfahren umfassend:
Schneiden eines planaren Materialbogens (910), um eine Vielzahl von linearen Streben (214) mit einem zentralen Strebenkreuzungspunkt (211) zu bilden;
Schneiden der Vielzahl von radialen Ausschnitten; und
Formen einer ungefähr sphäroiden oder abgeplattet sphäroiden Form mit den linearen Streben.

## Revendications

1. Ensemble panier extensible (38) pour une sonde médicale, comprenant :
une structure unitaire unique comprenant une pluralité de branches (214) convergeant au niveau d'une intersection centrale de branches (211), l'intersection centrale de branches étant positionnée sur un axe longitudinal de l'ensemble panier expansible au niveau d'une extrémité distale de celui-ci ; et
une pluralité de découpes radiales (212), chaque découpe radiale définissant une ouverture dans chacune de la pluralité de branches à proximité de l'intersection centrale de branches de sorte que chaque ouverture s'étend sur une longueur le long de chaque branche en s'éloignant de l'intersection centrale de branches, **caractérisée en ce que** chaque découpe comprend une découpe en forme de têtard (224), la découpe en têtard comporte une partie de tête (212B) contiguë à une circonférence d'un premier cercle virtuel (213) avec un premier rayon (r1) disposé autour de l'axe longitudinal, la partie de tête définissant une zone négative approximant un deuxième cercle virtuel avec un deuxième rayon (r2), la partie de tête étant reliée à une partie de queue fendue (212C) s'étendant sur une première longueur (L1) le long de la branche et contiguë à une circonférence intérieure d'un troisième cercle virtuel (215) ayant un troisième rayon (r3).

2. Ensemble panier expansible selon la revendication 1, dans lequel la première longueur (L1) de la partie de queue fendue est approximativement 6 à 10 fois celle de la longueur du rayon (r1) du premier cercle virtuel.

3. Ensemble panier expansible selon la revendication 2, comprenant en outre une découpe disposée sur l'axe longitudinal pour définir une zone négative centrale (A0) d'une ouverture centrale approximant un cercle central incluant un rayon central (r0) inférieur au premier rayon (r1).

4. Ensemble panier expansible selon la revendication 3, dans lequel la surface négative (A0) de chacune des découpes en forme de têtard comprend approximativement 0,2 mm carré tandis que la surface négative de l'ouverture centrale (212A) est approximativement 0,05 mm carré de sorte qu'une surface négative totale définie par toutes les découpes est approximativement 1,4 mm carré.

5. Ensemble panier expansible selon la revendication 3, dans lequel le rayon de vide central (r0) comprend approximativement 0,13 mm, le deuxième rayon (r2) comprend approximativement 0,2 mm, et le premier rayon (r1) comprend approximativement 0,23 mm.

6. Ensemble panier extensible (38) pour une sonde médicale, comprenant :
une structure unitaire unique comprenant une pluralité de branches (214) convergeant au niveau d'une intersection centrale de branches (211), l'intersection centrale de branches étant positionnée sur un axe longitudinal de l'ensemble panier expansible au niveau d'une extrémité distale de celui-ci ;
une pluralité de découpes radiales (212), chaque découpe radiale définissant une ouverture dans chacune de la pluralité de branches à proximité de l'intersection centrale de branches de sorte que chaque ouverture s'étend sur une longueur le long de chaque branche en s'éloignant de l'intersection centrale de branches ; et
**caractérisé en ce qu'il** comprend en outre une ouverture centrale (212A) disposée sur l'intersection de branche centrale, dans lequel la pluralité de découpes radiales sont séparées de l'ouverture centrale par une partie de l'intersection centrale de branches.

7. Ensemble panier expansible selon la revendication 6, dans lequel l'ouverture centrale comprend une surface d'environ 0,01 mm carré à environ 0,4 mm carré, éventuellement dans lequel chacune de la pluralité de découpes radiales comporte une surface d'environ 0,1 mm carré à environ 0,55 mm carré et chacune des branches comporte une première partie à proximité de l'intersection centrale de branches avec une première largeur, une deuxième partie à proximité de la première partie avec une deuxième largeur inférieure à la première partie, et une troisième partie à proximité de la deuxième partie avec une troisième largeur qui est supérieure à la première largeur et supérieure à la deuxième largeur, en outre éventuellement dans lequel la découpe définit une découpe en forme de comète (224) avec une partie de tête (212B) avec une queue effilée fendue (212C) s'étendant jusqu'à la partie proximale de chaque branche, encore en outre éventuellement dans lequel la première largeur est d'environ 0,15 mm à environ 0,5 mm, la deuxième largeur est d'environ 0,05 mm à environ 0,35 mm, et la troisième largeur est d'environ 0,3 mm à environ 0,7 mm, et la troisième partie de chaque branche comprend une fente de fixation d'électrode conçue pour recevoir une électrode, la fente de fixation d'électrode divise chaque branche en deux largeurs mineures d'environ 0,05 mm à environ 6 mm.

8. Ensemble panier extensible (38) pour une sonde médicale, comprenant :
une structure unitaire unique comprenant une pluralité de branches (214) convergeant au niveau d'une intersection centrale de branches (211), l'intersection centrale de branches étant positionnée sur un axe longitudinal de l'ensemble panier expansible au niveau d'une extrémité distale de celui-ci ; et
une pluralité de découpes radiales (212), chaque découpe radiale définissant une ouverture dans chacune de la pluralité de branches à proximité de l'intersection centrale de branches de sorte que chaque ouverture s'étend sur une longueur le long de chaque branche en s'éloignant de l'intersection centrale de branches, **caractérisée en ce que** chaque découpe comprend deux découpes en forme de goutte d'eau fixées au niveau de leurs parties étroites pour définir une seule découpe sur chaque branche.

9. Ensemble panier expansible selon la revendication 8, chaque découpe incluant une surface d'environ 0,4 mm carré.

10. Ensemble panier extensible (38) pour une sonde médicale, comprenant :
une structure unitaire unique comprenant une pluralité de branches (214) convergeant au niveau d'une intersection centrale de branches (211), l'intersection centrale de branches étant positionnée sur un axe longitudinal de l'ensemble panier expansible au niveau d'une extrémité distale de celui-ci ; et
une pluralité de découpes radiales (212), chaque découpe radiale définissant une ouverture dans chacune de la pluralité de branches à proximité de l'intersection centrale de branches de sorte que chaque ouverture s'étend sur une longueur le long de chaque branche en s'éloignant de l'intersection centrale de branches,
dans lequel chaque branche comprend deux parties de liaison (218), dans lequel la découpe radiale forme les deux parties de liaison (218) dans chaque branche,
**caractérisé en ce que** chaque branche comprend en outre une partie fendue s'étendant à l'écart des parties de liaison et de l'extrémité distale du panier, dans lequel i) la partie fendue de chaque branche comprend une fente (232) qui divise chaque branche en deux largeurs mineures (W8) dont chacune comprend une largeur d'approximativement 0,1 mm à approximativement 6 mm, ou ii) la partie fendue de chaque branche comprend une fente (232) qui divise chaque branche en deux parties mineures, la fente comprenant une largeur (W9) d'approximativement 0,05 mm à approximativement 0,55 mm et l'ouverture centrale comporte un rayon d'approximativement 0,4 mm à approximativement 1,2 mm, éventuellement dans lequel chaque découpe radiale comprend une forme d'ellipse au niveau d'une extrémité la plus éloignée de l'ouverture centrale et la forme d'ellipse comporte une longueur d'environ 0,20 mm à environ 0,55 mm et une largeur d'environ 0,1 mm à environ 0,45 mm.

11. Ensemble panier expansible pour une sonde médicale, comprenant :
une seule structure unitaire qui comporte une pluralité de branches convergeant au niveau d'une intersection centrale de branches, l'intersection centrale de branches étant positionnée sur un axe longitudinal de l'ensemble panier expansible au niveau d'une extrémité distale de celui-ci, **caractérisé par** la pluralité de branches convergeant au niveau de l'intersection centrale de branches selon un motif en spirale, dans lequel chacune de la pluralité de branches comporte une première largeur (W7) s'étendant à l'écart de l'extrémité distale et une seconde largeur (W4) associée au motif en spirale et plus étroite que la première largeur.

12. Ensemble panier expansible selon la revendication 11, dans lequel la première largeur est d'approximativement 0,1 mm à approximativement 1,0 mm, la seconde largeur est d'approximativement 0,05 mm à approximativement 0,65 mm, et chaque branche comprend une largeur effilée disposée entre les première et seconde largeurs.

13. Procédé de fabrication de l'ensemble panier selon l'une quelconque des revendications 1-10, le procédé comprenant :
la découpe d'une feuille plane de matériau (910) pour former une pluralité de branches linéaires (214) ayant une intersection centrale de branches (211) ;
la découpe de la pluralité de découpes radiales ; et
la formation d'une forme approximativement sphéroïde ou sphéroïde aplatie avec les branches linéaires.
